# NOUVEAU FASCICULE DE BREVET EUROPEEN

(11) **EP 0 914 079 B2**
(45) Date de publication et mention de la décision concernant l'opposition: **01.07.2009**
(45) Mention de la délivrance du brevet: 14.11.2001
(21) Numéro de dépôt: 97927251.5
(22) Date de dépôt: 06.06.1997
(51) Int. Cl.: A61K 8/895, A61K 8/898, A61K 8/899, A61Q 5/02, A61Q 5/12

(54) **COMPOSITIONS COSMETIQUES DETERGENTES A USAGE CAPILLAIRE COMPRENANT DES SILICONES**
SILIKONHALTIGES HAARWASCHMITTEL
HAIR SHAMPOO COMPRISING SILICONE COMPOUNDS

(30) Priorité: 07.06.1996 FR 9607192
(43) Date de publication de la demande: 12.05.1999
(73) Titulaire: L'ORÉAL, 75008 Paris (FR)
(72) Inventeur: DECOSTER, Sandrine, F-93800 Epinay sur Seine (FR)
(74) Mandataire: Dossmann, Gérard
(86) Numéro de dépôt international: PCT/FR1997/001007
(87) Numéro de publication internationale: WO 1997/046210

(56) Documents cités:
- EP-A- 0 412 704
- EP-A- 0 412 707
- WO-A-95/05800

## Description

La présente invention concerne de nouvelles compositions cosmétiques à propriétés améliorées destinées simultanément au nettoyage, au conditionnement et au coiffage des cheveux, et comprenant, dans un support cosmétiquement acceptable, une base lavante constituée de tensioactifs à pouvoir détergent dans laquelle sont également présents à titre d'agents conditionneurs, des polymères cationiques en association avec des silicones particulières. L'invention concerne aussi l'utilisation desdites compositions dans l'application cosmétique susmentionnée.

Pour le nettoyage et/ou le lavage des cheveux, l'utilisation de compositions capillaires détergentes (ou shampooings) à base essentiellement d'agents tensio-actifs classiques de type notamment anioniques, non-ioniques et/ou amphothères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entrainer à la longue sur la fibre capillaire des dommages plus ou moins marqués liés en particulier à l'élimination progressive des lipides ou protéïnes contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abimés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, qui confèrent en effet aux cheveux lavés, secs ou mouillés, une facilité de démêlage, une douceur et un lissage nettement accrus par rapport à ce qui peut être obtenu avec les compositions nettoyantes correspondantes qui en sont exemptes.

Par ailleurs, depuis quelque temps, on cherche à obtenir des shampooings conditionneurs qui soient capables d'apporter aux cheveux lavés non seulement les propriétés cosmétiques mentionnées ci-avant mais aussi, à un degré plus ou moins poussé, des propriétés de coiffage, de volume, de mise en forme et de tenue. Ces derniers shampooings lavants à propriétés cosmétiques générales améliorées sont souvent dénommés, par simplicité, "shampooings à effets coiffants", expression qui sera d'ailleurs reprise dans la suite de l'exposé.

La demande WO95/05800 décrit une composition de soin pour améliorer le temps de séchage comprenant un polymère adhésif greffé polysiloxane, un solvant volatile pour ledit polymère, un agent non volatile choisi parmi les silicones.

Toutefois, et malgré les progrès réalisés récemment dans le domaine des shampooings à effets coiffants à base de polymères cationiques, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoqués ci-avant, de meilleures performances.

La présente invention vise à la satisfaction d'un tel besoin.

Ainsi, à la suite d'importantes recherches menées sur la question, il a maintenant été trouvé par la Demanderesse, de façon totalement inattendue et surprenante, qu'en utilisant (A) une base lavante, comprenant un ou plusieurs tensioactifs choisis parmi les tensioactifs anioniques, amphotères non-ioniques. zwittérioniques, cationiques et leurs mélanges et (B) un système conditionneur comprenant au moins un polymère cationique et au moins un mélange de silicones particulières convenablement sélectionnées, tels que définis ci-après, il est possible d'obtenir des compositions détergentes présentant d'excellentes propriétés cosmétiques, en particulier en particulier la facilité de coiffage, le maintien, la nervosité et le volume des cheveux traités et ceci tout en conservant leur bon pouvoir lavant intrinsèque.

Toutes ces découvertes sont à la base de la présente invention.

Ainsi, selon la présente invention, il est maintenant proposé de nouvelles compositions capillaires détergentes et conditionnantes comprenant, dans un milieu cosmétiquement acceptable, (A) une base lavante et (B) un système conditionneur comprenant au moins un polymère cationique et un mélange d'au moins un polymère siliconé greffé comprenant une portion polysiloxane et une portion constituée d'une chaîne organique non-siliconée, l'une des deux portions constituant la chaîne principale du polymère, l'autre étant greffée sur ladite chaîne principale et au moins une silicone aminée, les polymères siliconés greffés étant choisis parmi les polymères, à squelette organique non-siliconé, constitué d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane ou les polymères à squelette polysiloxanique greffé par des monomères non-siliconés susceptibles d'être obtenus par copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente.

L'invention a également pour objet l'utilisation en cosmétique des compositions ci-dessus pour le nettoyage, le conditionnement et/ou le coiffage des cheveux.

Mais d'autres caractéristiques, aspects et avantages de l'invention apparaitront encore plus clairement à la lecture de la description qui va suivre ainsi que des exemples concrets, mais nullement limitatifs, destinés à l'illustrer.

Comme indiqué précédemment, les constituants essentiels rentrant dans la composition des produits capillaires de l'invention sont (A) une base lavante, (B) un système conditionneur comprenant (i) le ou les polymères cationiques, et (ii) un mélange d'au moins un polymère siliconé greffé et d'au moins une silicone aminée.

### A- BASE LAVANTE :

Les compositions conformes à l'invention comprennent nécessairement une base lavante, généralement aqueuse.

Le ou les tensioactifs formant la base lavante peuvent être indifféremment choisis, seuls ou en mélanges, au sein des tensioactifs anioniques, amphotères, non ioniques, zwittérioniques et cationiques.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 4 % à 50 % en poids, de préférence de 10 % à 35 % en poids, et encore plus préférentiellement de 12 % à 25 % en poids, du poids total de la composition finale.

Les tensioactifs convenant à la mise en oeuvre de la présente invention sont notamment les suivants :

### (i) Tensioactif(s) anionique(s) :

Leur nature ne revet pas, dans le cadre de la présente invention, de caractère véritablement critique.

Ainsi, à titre d'exemple ce tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique. les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante :

R₁―(OC₂H₄)ₙ―OCH₂COOA (1)

dans laquelle :

R₁ désigne un groupement alkyle ou alkaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,

A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Parmi les tensioactifs anioniques, on préfère utiliser les sels d'alkylsulfates et les sels d'alkyléthersulfates

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s):

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂-CONHCH₂CH₂ -N(R₃)(R₄)(CH₂COO⁻) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₂'-CONHCH₂CH₂-N(B)(C) (3)

dans laquelle :
B représente -CH₂CH₂OX', C représente -(CH₂)_{z}-Y'. avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate vendu sous la dénomination commerciale MIRANOL C2M concentré par la Société MIRANOL.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

On notera que les tensioactifs cationiques, dont l'utilisation n'est pas exclue, ne constituent pas des tensioactifs préférés *pour* la mise en oeuvre de la présente invention.

### B- SYSTEME CONDITIONNEUR

### (i) Polymère(s) cationique(s) :

Les compositions conformes à l'invention comprennent en outre nécessairement un polymère cationique.

Les agents conditionneurs de type polymères cationiques utilisables conformément à la présente invention peuvent être choisis parmi tous ceux déja connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire en nombre comprise entre 500 et 5.10⁶ environ, et de préférence comprise entre 10³ et 3.10⁶ environ.

Parmi les polymères cationiques, on peut citer plus particulièrement les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société Maybroook et dénommnés dans le dictionnaire CTFA "Steartrimonium Hydrolyzed Collagen";
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le "Croquat L" dont les groupements ammonium quaternaires comportent un groupement alkyle en C₁₂ ;
- le "Croquat M" dont les groupements ammonium quaternairees comportent des groupements alkyle en C₁₀-C₁₈;
- le "Croquat S" dont les groupements ammonium quaternaires comportent un groupement alkyle en C₁₈ ;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule : 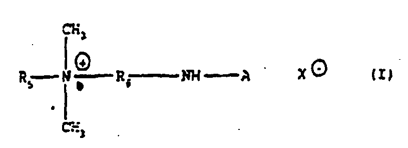 dans laquelle X⁻ est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société Index. sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées, on peut citer celles commercialisées par la Société Croda sous les dénominations "Hydrotriticum WQ ou QM", appelées dans le aictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) Les copolymères vinylpyrrolidone-acrylate ou -méthacrylate de dialkylaminoalkyle quaternisés ou non, tels que les produits vendus sous la dénomination "Gafquat" par la Société ISP, comme par exemple Gafquat 734, 755 ou HS100 ou bien le produit dénommé "Copolymère 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bishalohydrine, un bis-azétidinium, une bis-haloacyldiamine, un bis-halogénure d'alkyle ou encore par un oligomère résultant' de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la scciété Hercules dans le cas du copolymère d'acide adipique/époxypropyl/diéthylène-triamine.
(9) les cyclohomopolymères de méthyl diallyl amine ou de diméthyl diallyl ammonium tels que les homopolymères comportant comme constituant principal de la chaîne des motifs répondant aux formules (VI) ou (VI') : 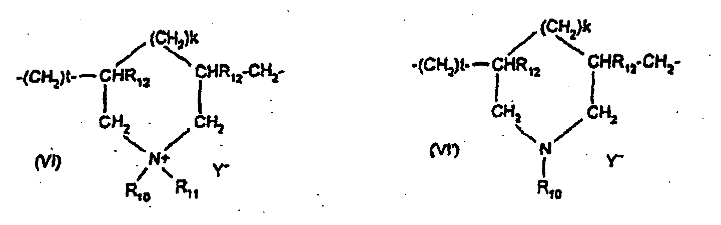 formules dans lesquelles k et t sont égaux à 0 ou 1, la somme k + t étant égale à 1 ; R12 désigne un atome d'hydrogène ou un radical méthyle ; R10 et R11, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R10 et R11 peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Merck.
(10) le polymère de diammonium quaternaire contenant des motifs récurants répondant à la formule : 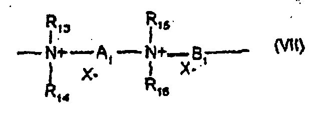 formule (VII) dans laquelle :
   R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un aikylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement

      (CH2n-CO-D-OC-(CH2)n-

      dans lequel D désigne :
      a) un reste de glycol de formule : -O-Z-O-, où Z désigne un radical hydrocarboné linéaire ou ramifié ou un groupement répondant à l'une des formules suivantes :

         -(CH2-CH2-O)x-CH2-CH2-

         -[CH2-CH(CH3)-O]y-CH2-CH(CH3)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent
         -CH₂-CH₂-S-S-CH₂-CH₂- :
      d) un groupement uréylène de formule : -NH-CO-NH- ;

De préférence, X⁻ est un anion tel que le chlorure ou le bromure.

Ces polymères ont une masse moléculaire en nombre généralement comprise entre 1000 et 100000.

Des polymères de ce type sont notamment décrits dans les brevets français 2.320.330, 2.270,846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) les polymères de polyammonium quaternaires constitués de motifs de formule (VIII): 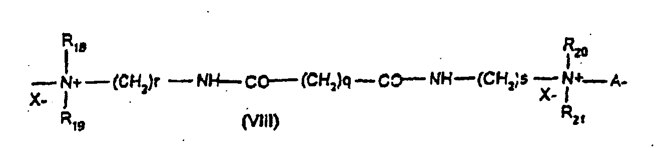 formule dans laquelle :
   R18, R19, R20 et R21, identiques ou différents, représentent un atome d'hydrogène ou un radical méthyle, éthyle, propyle, β-hydroxyethyle, β-hydroxypropyle ou -CH2CH2(OCH2CH2)pOH,
   où p est égal à 0 ou à un nombre entier compris entre 1 et 6, sous réserve que R18, R19, R20 et R21 ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH2-CH2-O-CH2-CH2-.
   De tels composés sont notamment décrits dans la demande de brevet EP-A-122 324.
   On peut par exemple citer parmi ceux-ci, les produits "Mirapol A 15", "Mirapol AD1", "Mirapol AZ1" et "Mirapol 175" vendus par la société Miranol.
(12) les homopolymères ou copolymères dérivés des acides acrylique ou méthacrylique et comportant des motifs : 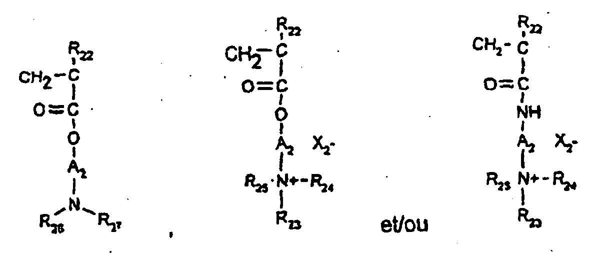 dans lesquels les groupements R22 désignent indépendamment H ou CH3,

les groupements A1 désignent indépendamment un groupe alcoyle linéaire ou ramifié de 1 à 6 atomes de carbone ou un groupe hydroxyalcoyle de 1 à 4 atomes de carbone,

les groupements R23, R24, R25, identiques ou différents, désignant indépendamment un groupe alcoyle de 1 à 18 atomes de carbone ouun radical benzyle,

les groupements R26 et R27 représentent unatome d'hydrogène ou un groupement alcoyle de 1 à 6 atomes de carbone,

X₂⁻ désigne un anion, par exemple méthosulfate ou halogénure, tel que chlorure ou bromure.

Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinyipyrrolidone ou des esters vinyliques.
(13) Les polymères quaternaires de vinylpyrrolidone et de vinylimidazole tels que par exemple les produits commercialisés sous les dénominations Luviquat FC 905, FC 550 et FC 370 par la société B.A.S.F..
(14) Les polyamines comme le Polyquart H vendu par HENKEL, référencé sous le nom de « POLYETHYLENEGLYCOL (15) TALLOW POLYAMINE » dans le dictionnaire CTFA.
(15) Les polymères réticulés de chlorure de méthacryloyloxyéthyltriméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copofyrnérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE SC 92 » par la Société ALLIED COLLOIDS. On peut également utiliser un homopofymère réticulé du chlorure de méthacryloyloxyéthyl triméthylammonium contenant environ 50 % en poids de l'homopolymère dans de l'huile minérale. Cette dispersion est commercialisée sous le nom de « SALCARE SC 95 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Selon l'invention, on peut utiliser plus particulièrement les polymères choisis parmi le Mirapol, le composé de formule (VII) dans laquelle R13, R14, R15 et R16 représentent le radical méthyle, A1 représente le radical de formule -(CH2)3- et B1 représente le radical de formule -(CH2)6- et X⁻ représente l'anion chlorure (nommé ultérieurement Mexomère PO) et le composé de formule (VII) dans laquelle R13 et R14 représentent le radical éthyle, R15 et R16 représentent le radical méthyle, A1 et B1 représentent le radical de formule -(CH2)3- et X représente l'anion bromure (nommé ultérieurement Mexomère PAK).

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères, en particulier les copolymères du chlorure de diméthyldiallylammonium et d'acrylamide, vendus sous les dénominations « MERQUAT 550 » et « MERQUAT S » par la Société MERCK, les polysaccharides cationiques et plus particulièrement la gomme de guar modifiée par du chlorure de 2,3-époxypropyl triméthylammonium vendue sous la dénomination « JAGUAR C13S » par la Société MEYHALL.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

### (ii)- Mélange de silicones

Selon une caractéristique essentielle des compositions capillaires détergentes conformes à l'invention, ces dernières contiennent en outre un mélange d'au moins un silicone spécifique de type polymères siliconés greffés et d'au moins une silicone aminée (différente de la précédente).

### (1) Polymères siliconés greffés

Selon une caractéristique essentielle des compositions capillaires détergentes conformes à l'invention, ces dernières contiennent en outre au moins un polymère siliconé greffé.

Dans tout ce qui suit ou qui précède, on entend désigner par silicone ou polysiloxane, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxy ou acyloxyalkyls, les radicaux hydroxyalkylamino ou aminoalkyls, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Dans ce qui suit, on entend désigner par «macromère polysiloxane», en conformité avec l'acceptation générale, tout monomère contenant dans sa structure une chaîne polymère du type polysiloxane.

Les monomères organiques non-siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture ce cycle tels que ceux du type oxazoline ou caprolactone.

Les polymères à squelette organique non-siliconé greffé par des monomères contenant un polysiloxane conformes à la présente invention peuvent être obtenus selon tout moyen connu de l'homme de l'art, en particulier par réaction entre (i) un macromère polysiloxane de départ correctement fonctionnalisée sur la chaîne polysiloxanique et (ii) un ou plusieurs composés organiques non-siliconés, eux-mêmes correctement fonctionnalisés par une fonction qui est capable de venir réagir avec le ou les groupements fonctionnels portés par ladite silicone en formant une liaison covalente ; un exemple classique d'une telle réaction est la réaction radicalaire entre un groupement vinylique porté sur une des extrémités de la silicone avec une double liaison d'un monomère à insaturation éthylénique de la chaîne principale.

Les polymères à souelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0412704, EP-A-0412707, EP-A-0640105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Une famille particulière de polymères siliconés greffés convenant pour la réalisation de la présente invention est constituée par les copolymères greffés siliconés comprenant :
a) de 0 à 98% en poids d'au moins un monomère(A) lipophile de faible polarité lipophile à insaturation éthylénique, polymérisable par voie radicalaire ;
b) de 0 à 98% en poids d'au moins un monomère (B) hydrophile polaire à insaturation éthylénique, copolymérisable avec le ou les monomères du type (A)
c) de 0,01 à 50 % en poids d'au moins un macromère polysiloxane (C) de formule générale :

   X(Y)ₙSi(R)₃₋ₘ Zₘ (I)

   où :
   X désigne un groupe vinylique copolymérisable avec les monomères (A) et (B) ;
   Y désigne un groupe de liaison divalent ;
   R désigne un hydrogène, un alkyle ou un alcoxy en C₁-C₆, un aryle C₆-C₁₂ ;
   Z désigne un motif polysiloxane monovalent ayant un poids moléculaire moyen en nombre d'au moins 500 ;
   n est 0 ou 1 et m est un entier allant de 1 à 3 ; les pourcentages étant calculés par rapport au poids total des monomères (A), (B) et (C).

Ces polymères sont décrits ainsi que leurs procédés de préparation dans les brevets US 4,693,935, US 4,728,571 et US 4,972,037 et les demandes de brevet EP-A-0412704, EP-A-0412707, EP-A-0640105. Ils ont un poids moléculaire moyen en nombre de préférence allant de 10.000 à 2.000.000 et de préférence une température de transition vitreuse Tg ou une température de fusion cristalline Tm d'au moins -20°C.

On peut citer comme exemples de monomères lipophiles (A), les esters d'acide acrylique ou méthacrylique d'alcools en C₁-C₁₈ ; le styrène ; les macromères polystyrène ; l'acétate de vinyle ; le propionate de vinyle ; l'alpha-méthylstyrène ; le tertio-butylstyrène ; le butadiène ; le cyclohexadiène ; l'éthylène ; le propylène ; le vinyltoluène ; les esters d'acide acrylique ou méthacrylique et de 1,1-dihydroperfluoroalcanol ou de ses homologues ; les esters d'acide acrylique ou méthacrylique et de oméga-hydrydofluroalcanol ; les esters d'acide acrylique ou méthacrylique et de fluoroalkylsulfoamido-alcool; les esters d'acide acrylique ou méthacrylique et d'alcool fluoroalkylique ; les esters d'acide acrylique ou méthacrylique et de fluroéther d'alcool ; ou leurs mélanges.

Les monomères (A) préférentiels sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyie, le méthacrylate de méthyle, le 2-(N-méthyl perflurooctane sulfonamido)-éthylacrylate; le 2-(N-butylperflurooctane sulfonamido)-éthylacrylate et leurs mélanges.

On peut citer comme exemples de monomères polaires (B), l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butylacrylamide, l'acide maleique, l'anhydride maléique et leurs demi-esters, les (méth)acrylates hydroxyalkylés, le chlorure de diallyldiméthylammonium, la vinyipyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinylcaprolactame ou leurs mélanges. Les monomères (B) préférentiels sont choisis dans le groupe constitué par l'acide acrylique, le N,N-diméthylacrylamide, le méthacrylate de diméthylaminoéthyle, le méthacrylate de diméthylaminoéthyle quaternisé, la vinylpyrrolidone et leurs mélanges.

Les macromères polysiloxane (C) de formule (I) préférentiels sont choisis parmi ceux répondant à la formule générale suivante (II) : 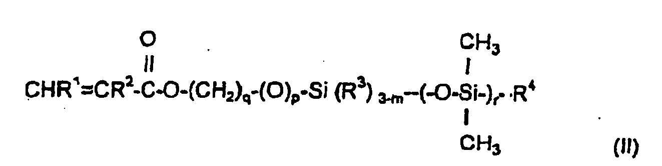 dans laquelle :
R¹ est hydrogène ou -COOH (de préférence hydrogène) ;
R² est hydrogène, méthyle ou -CH₂COOH (de préférence méthyle) ;
R³ est alkyle, alcoxy ou alkylamino en C₁-C₆, aryle en C₆-C₁₂ ou hydroxyle (de préférence méthyle) ;
R⁴ est alkyle, alcoxy ou alkylamino en C₁-C₆, aryle en C₆-C₁₂ ou hydroxyle (de préférence méthyle) ;
q est un entier de 2 à 6 (de préférence 3) ;
p est 0 ou 1 ;
r est un nombre entier de 5 à 700 ;
m est un entier allant de 1 à 3 (de préférence 1) ;

On utilise plus particulièrement les macromères polysiloxanes de formule : 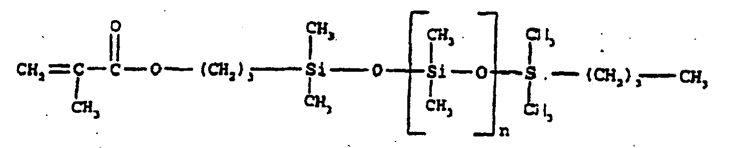 avec n étant un nombre allant de 5 à 700.

Un mode particulier de réalisation de l'invention consiste à utiliser un copolymère susceptible d'être obtenu par polymérisation radicalaire à partir du mélange de monomères constitué par :
a) 60% en poids d'acrylate de tertiobutyle ;
b) 20% en poids d'acide acrylique ;
c) 20% en poids de macromère siliconé de formule : 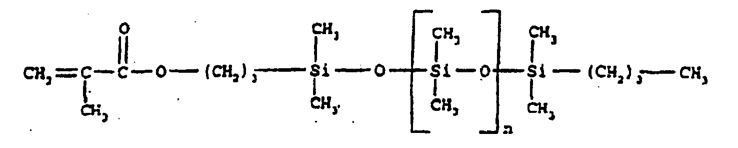 avec n étant un nombre allant de 5 à 700 ; les pourcentages en poids étant calculés par rapport au poids total des monomères,

Un autre mode particulier de réalisation de l'invention consiste à utiliser un copolymère susceptible d'être obtenu par polymérisation radicalaire à partir du mélange de monomères constitué par :
a) 80% en poids d'acrylate de tertiobutyle ;
b) 20% en poids de macromère siliconé de formule : 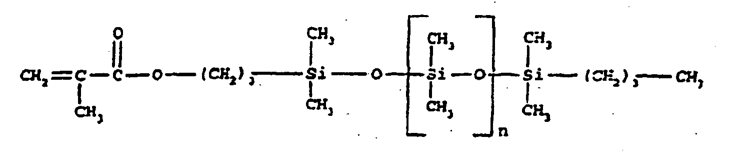 avec n étant un nombre allant de 5 à 700 ; les pourcentages en poids étant calculés par rapport au poids total des monomères.

Une autre famille particulière de polymères siliconés convenant pour la réalisation de la présente invention est constituée par les copolymères greffés siliconés susceptibles d'être obtenus par extrusion réactive d'un macromère polysiloxane ayant une fonction réactive terminale sur un polymère du type polyoléfine comportant des groupes réactifs susceptibles de réagir avec la fonction terminale du macromère polysiloxane pour former une liaison covalente permettant le greffage de la silicone sur la chaine principale de la polyoléfine.

Ces polymères sont décrits ainsi que leur procédé de préparation dans la demande de brevet WO 95/00578.

Les polyoléfines réactives sont choisies de préférence parmi les polyéthylènes ou les polymères de monomères dérivés de l'éthylène tels que propylène, styrène, alkylstyrène, butylène, butadiène, les (méth)acrylates, les esters de vinyle ou équivalents, comportant des fonctions réactives susceptibles de réagir avec la fonction terminale du macromère polysiloxane. Ils sont choisis plus particulièrement parmi les copolymères d'éthylène ou de dérivés d'éthylène et de monomères choisis parmi ceux comportant une fonction carboxyiique tels que l'acide (méth)acrylique ; ceux comportant une fonction anhydride d'acide tels que l'anhydride de l'acide maléique ; ceux comportant une fonction chlorure d'acide tels que le chlorure de l'acide (méth)acrylique ; ceux comportant une fonction ester tels que les esters de l'acide (méth)acrylique ; ceux comportant une fonction isocyanate.

Les macromères siliconés sont choisis de préférence parmi les polysiloxanes comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires et plus particulièrement parmi ceux répondant à la formule générale (III):

T-(CH₂)ₛ-Si-[-(OSiR⁵R⁶)ₜ-R⁷]_{y} (III)

dans laquelle T est choisi dans le groupe constitué par NH2, NHR', une fonction époxy, OH, SH ; R⁵, R⁶, R⁷ et R', indépendamment, désignent un alkyle en C₁-C₆, phenyle, benzyle, ou alkylphényle en C₆-C₁₂, hydrogène ; s est un nombre de 2 à 100 ; t est un nombre de 0 à 1000 et y est un nombre de 1 à 3. Ils ont un poids moléculaire moyen en nombre de préférence allant de 5.000 à 300.000, plus préférentiellement de 8.000 à 200.000 et plus particulièrement de 9.000 à 40.000.

Selon la présente invention, le ou les polymères siliconés greffés, à squelette polysiloxanique greffé par des monomères organiques non-siliconés comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Les polymères à squelette polysiloxanique greffé par des monomères organiques non-siliconés selon l'invention peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thiofonctionnels -SH avec ces mêmes groupements vinyliques.

Des exemples de polymères à squelette polysiloxanique greffé par des monomères organiques non-silicones convenant à la mise en oeuvre de la présente invention, ainsi que leur mode particulier de préparation, sont notamment décrits dans les demandes de brevets EP-A-0582152, WO 93/23009 et WO 95/03776 dont les enseignements sont totalement inclus dans la présente description à titre de références non limitatives.

Selon un mode particulièrement préféré de réalisation de la présente invention, le polymère silicosé, à squelette polysiloxanique greffé par des monoméres organiques non-siliconés, mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement thio-fonctionnel capable de venir réagir sur lesdites insaturations éthyléniques desdits monomères non-siliconés en formant une liaison covalente.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélanges, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le (math)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

Une famille de polymères siliconés à squelette polysiloxanique greffé par des monomères organiques non-siliconés convenant particulièrement bien à la mise en oeuvre de la présente invention est constituée par les polymères siliconés comportant dans leur structure le motif de formule (I) suivant : 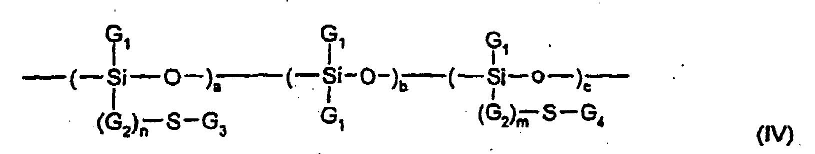 dans lequel les radicaux G₁ , identiques ou différents, représentent l'hydrogène ou un radical alkyle en C₁-C₁₀ ou encore un radical phényle ; les radicaux G₂ , identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (IV) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle(C₁-C₁₀), de préférence le (méth)acrylate d'isobutyle ou de méthyle.

Des exemples de polymères siliconés greffés répondant à la formule (IV) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés greffés répondant à la formule (IV) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non-siliconés de l'invention varie de 10 000 à 1 000 000 environ, et encore plus préférentiellement de 10 000 à 100 000 environ.

Les polymères greffés siliconés de l'invention sont utilisés de préférence en une quantité allant de 0,01 à 20% en poids du poids total de la composition. Plus préférentiellement, cette quantité varie de 0,1 à 15% en poids et encore plus préférentiellement de 0,5 à 10% en poids.

### (2)- Silicone(s) aminée(s)

Selon l'invention, on désigne par silicone aminée toute silicone comportant au moins une amine primaire, secondaire, tertiaire ou un groupement ammonium quaternaire. On peut ainsi citer :
(a) les polysiloxanes dénommés dans le dictionnaire CTFA "amodiméthicone" et répondant à la formule : 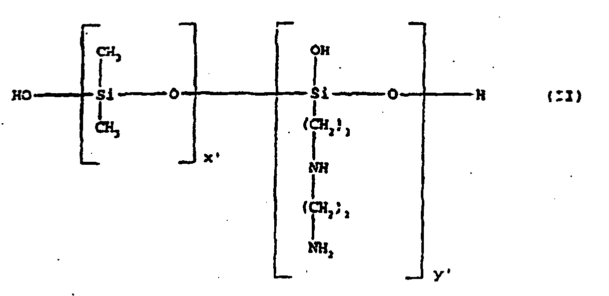 dans laquelle x' et y' sont des nombres entiers dépendant du poids moléculaire, généralement tels que ledit poids moléculaire en nombre est compris entre 5 000 et 500 000 environ ;
(b) les polymères cationiques siliconés répondant à la formule :

   R'ₐG₃₋ₐ-Si(OSiG₂)ₙ-(OSiG_{b}R'_{2-b})ₘ-O-SiG₃₋ₐ-R'ₐ (III)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R' est un radical monovalent de formule -C_{q}H_{2q}L dans laquelle q est un nombre de 2 à 8 et L est un groupement aminé éventuellement quatemisé choisi parmi les groupements :
      -NR"-CH₂-CH₂-N'(R")₂
      -N(R")₂
      -N^{⊕}(R")₃ A⁻
      -N^{⊕}(R")₃ A⁻
      -N^{⊕}(R")₃A⁻
      -N(R")-CH₂-CH₂-N^{⊕}R"H₂A⁻,
      dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
      Un produit correspondant à cette définition est le polymère dénommé "triméthylsilylamodiméthicone", répondant à la formule : 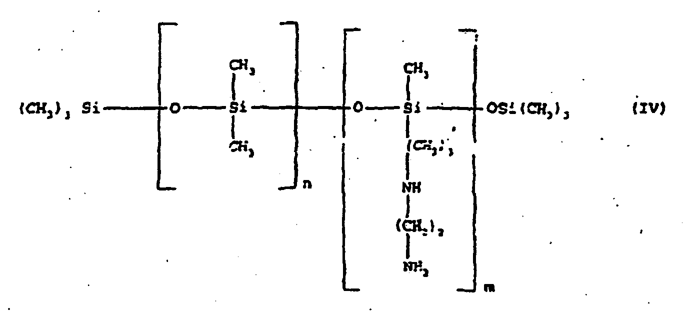 dans laquelle n et m ont les significations données ci-dessus (cf formule III).
      De tels polymères sont décrits par exemple dans la demande de brevet EP-A-95238.
(c) les polymères cationiques siliconés répondant à la formule : 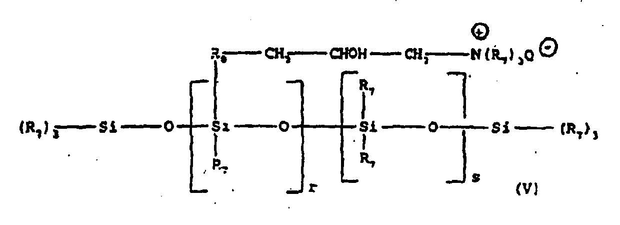 dans laquelle
   R₇ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₈ représente un radical hydrocarboné divalent, noatmment un radical alkylène en C₁-C₁₈ ou un radical alkylèneoxy divalent en C₁-C₁₈, par exemple en C₁-C₈;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

De tels polymères sont décrits plus particulièrement dans le brevet US 4 185 087.

Un polymère entrant dans cette classe est le polymère vendu par la Société Union Carbide sous la dénomination "Ucar Silicone ALE 563.

Lorsque ces polymères siliconés sont mis en oeuvre, une forme de réalisation particulièrement intéressante est leur utilisation conjointe avec des agents de surface cationiques et/ou non ioniques. On peut utiliser par exemple le produit vendu sous la dénomination "Emulsion Cationique DC 929" par la Société Dow Corning qui comprend, outre l'amodiméthicone, un agent de surface cationique comprenant un mélange de produits répondant à la formule : 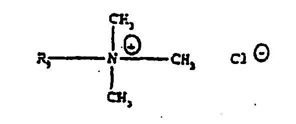 dans lequel R₉ désigne des radicaux alcényle et/ou alcoyle ayant de 14 à 22 atomes de carbone, dérivés des acides gras du suif,
en association avec un agent de surface non ionique de formule :

C₉H₁₉-C₆H₄-(OC₂H₄)₁₀-OH

connu sous la dénomination "Nonoxynol 10".

On peut également utiliser par exemple le produit vendu sous la dénomination "Emulsion Cationique DC 939" par la Société Dow Corning qui comprend, outre l'amodiméthicone, un agent de surface cationique le chlorure de triméthyl cétyl ammonium en association avec un agent de surface non ionique le tridéceth-12.

Un autre produit commercial utilisable selon l'invention est le produit vendu sous la dénomination "Dow Corning Q2 7224" par la Société Dow Corning comportant en association le triméthylsilylamodiméthicone de formule (IV), un agent de surface non ionique de formule : C₈H₁₇-C₆H₄-(OCH₂CH₂)ₙ-OH où n = 40 dénommé encore octoxynol-40, un autre agent de surface non ionique de formule C₁₂H₂₅-(OCH₂-CH₂)ₙ-OH où n = 6 encore dénommé isolaureth-6, et du glycol.

Les compositions capillaires conformes à l'invention renferment les silicones aminées définies ci-dessus à des teneurs pondérales qui peuvent être comprises entre 0,05 % et 10 %, de préférence entre 0,1 % et 5 % et encore plus préférentiellement entre 0,2 % et 3%, par rapport au poids total de la composition.

Le véhicule, ou support, des compositions détergentes selon l'invention est de préférence de l'eau ou une solution hydroalcoolique d'un alcool inférieur tel que éthanol, isopropanol ou butanol.

Les compositions détergentes selon l'invention présentent un pH final généralement compris entre 3 et 10. De préférence, ce pH est compris entre 5,5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions détergentes selon l'invention peuvent bien entendu contenir en outre tous les adjuvants usuels rencontrés dans le domaine des shampooings, comme par exemple des parfums, des agents conservateurs, des sequestrants, des épaississants, des adoucissants, des modificateurs de mousse, des colorants, des agents nacrants, des agents hydratants, des agents antipélliculaires ou anti-séborrhéiques, des vitamines, des filtres solaires et autres.

Bien entendu, l'homme de l'art veillera à choisir ce ou ces éventuels composés complémentaires et/ou leurs quantités de manière telle que les propriétés avantageuses attachées intrinsèquement à l'association ternaire (base lavante + polymère cationique + deux silicones spécifiques) conforme à l'invention ne soient pas, ou substantiellement pas, altérées par la ou les adjonctions envisagées.

Ces compositions peuvent se présenter sous la forme de liquides plus ou moins épaissis, de crèmes ou de gel et elles conviennent principalement au lavage, au soin et/ou le coiffage des cheveux. Elles peuvent aussi se présenter sous la forme de lotions à rincer.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des fibres kératiniques telles que les cheveux consistant à appliquer sur lesdites fibres mouillées une quantité efficace d'une composition telle que définie ci-dessus, puis à effectuer un rinçage à l'eau après un éventuel temps de pause.

Comme indiqué précédemment, les compositions conformes à l'invention confèrent aux cheveux, après rinçage, un remarquable effet coiffant qui se manifeste notamment par une facilité de coiffage et de maintien, ainsi qu'un apport de volume et de légèreté, nettement améliorés.

Des exemples concrets, mais nullement limitatifs, illustrant l'invention vont maintenant être donnés.

### EXEMPLE 1

On a réalisé deux compositions de shampooing, l'une conforme à l'invention (composition A) et I' autre comparative (composition B) :

| | A Invention | B Comparatif |
|---|---|---|
| - Lauryléthersulfate de sodium (C12/C14 à 70/30) à 2,2 moles d'oxyde d'éthylène en solution aqueuse à 28% de MA (MA = matière active) | 14 gMA | 14 gMA |
| - Cocoylbétaïne à 32% MA(*) | 3,2 gMA | 3,2 gMA |
| - Polymère cationique (**) | 0,1 g | 0,1 g |
| - Silicone aminée (***) | 1,05 gMA | ― |
| - Polymère silicone greffé (****) | 2 gMA | 2 gMA |
| - Mélange de 1-hexadécyloxy cctadodécanol et d'alcool cétylique | 2,5 g | 2,5 g |
| - Monoisopropanolamide d'acides de coprah | 0,6 g | 0,6 g |
| - Acide citrique qs pH | 5,2 | 5,2 |
| - Eau déminéralisée qs | 100 g | 100 g |

| | | |
|---|---|---|
| (*) DEHYTON AB'30 de HENKEL (**) : gomme de guar modifiée par du chlorure de 2,3-époxypropyl triméthylammonium vendue sous la dénomination JAGUAR C13 S par la société RHONE POULENC (***) : Amodiméthicone vendue en émuision cationique à 35% de matière active sous la dénomination FLUID DC 939 par la société DOW CORNING (****) : Polydiméthyl/méthylsiloxane à groupements propyl thio 3 acide polyméthacrylique / polyméthacrylate d'isobutyle / acide méthacrylique, préneutralisé à l'ammoniaque et vendu en solution aqueuse à 10% de matière active sous la dénomination de VS 80 par la Société 3M. | | |

On effectue un shampooing en appliquant environ 12 g de la composition A sur des cheveux préalablement mouillés. On fait mousser le shampooing puis on rince abondamment à l'eau.
On procède selon le même mode opératoire que ci-dessus avec la composition comparative B.

Un panel d'experts évalue le démêlage des cheveux mouillés, le démêlage des cheveux séchés, la facilité de mise en forme, la douceur, le volume, la nervosité et le lissage des cheveux séchés.

Tous les experts indiquent une amélioration nette de ces propriétés pour les cheveux traités avec la composition A selon l'invention.

De plus, le séchage des cheveux traités avec la composition A est plus rapide.

### EXEMPLE 2

On a préparé un shampooing de composition suivante :
- Lauryléthersulfate de sodium (C12/C14
   à 70/30) à 2,2 moles d'oxyde d'éthylène
   en solution aqueuse à 28% de MA 14 gMA
- Cocoylbétaïne à 32% MA 3,2 gMA
- Polymère cationique 0,1 g
- Silicone aminée 1,05 gMA
- Polymère silicone greffé 0,5 gMA
- Mélange de 1-hexadécyloxy 2,5 g octadodécanol et d'alcool cétylique
- Monoisopropanolamide d'acides de 0,6 g coprah
- Amino-2 méthyl-2 propanol-1 0,125 g
- Acide citrique qs pH 5,2
- Eau déminéralisée qs 100 g

## Revendications

1. Composition capillaire détergente et conditionnante, **caractérisée par le fait qu'**elle comprend, dans un milieu cosmétiquement acceptable, (A) une base lavante comprenant un ou plusieurs tensioactifs choisis parmi des tensioactifs anioniques, amphotères, non anioniques , zwittérioniques, cationiques et leurs mélanges et (B) un système conditionneur comprenant au moins un polymère cationique et un mélange d'au moins une silicone aminée et d'au moins un polymère siliconé greffé comprenant une portion polysiloxane et une portion constituée d'une chaîne organique non-siliconée, l'une des deux portions constituant la chaîne principale du polymère, l'autre étant greffée sur ladite chaîne principale, les polymères siliconés greffés étant choisis parmi les polymères, à squelette organique non-siliconé, constitué d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromère polysiloxane ou les polymères à squelette polysiloxanique greffé par des monomères non-siliconés susceptibles d'être obtenus par copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente.

2. Composition selon la revendication 2, **caractérisée par le fait que** les monomères organiques non-siliconés constituant la chaîne principale du polymère siliconé greffé sont choisis dans le groupe constitué par des monomères à insaturation éthylènique polymérisables par voie radicalaire, des monomères polymérisables par polycondensation, des monomères à ouverture de cycle.

3. Composition selon l'une quelconque des revendications 1 à 2, **caractérisée par le fait qu'**elle contient au moins un polymère siliconé greffé comprenant :
a) de 0 à 98% en poids d'au moins un monomère (A) lipophile de faible polarité à insaturation éthylénique de faible polarité, polymérisable par voie radicalaire ;
b) de 0 à 98% en poids d'au moins un monomère (**B**) hydrophile polaire à insaturation éthylénique, copolymérisable avec le ou les monomères du type (**A**)
c) de 0,01 à 50 % en poids d'au moins un macromère polysiloxane (**C**) de formule générale :
X(Y)ₙSi(R)₃₋ₘZₘ (I)
où :
X désigne un groupe vinylique copolymérisable avec les monomères (**A**) et (**B**) ;
Y désigne un groupe de liaison divalent ;
R désigne un hydrogène, un alkyle ou un alcoxy en C₁-C₆, un aryle C₆-C₁₂ ;
Z désigne un motif polysiloxane monovalent ayant un poids moléculaire moyen en nombre d'au moins 500 ;
n est 0 ou 1 et m est un entier allant de 1 à 3 ; les pourcentages étant calculés par rapport au poids total des monomères (A), (B) et (C).

4. Composition selon la revendication 3, **caractérisée par le fait que** les monoméres lipophiles (A) sont choisis dans le groupe constitué par les esters d'acide acrylique ou méthacrylique d'alcools en C₁-C₁₈ ; le styrène ; les macromères polystyrène ; l'acétate de vinyle ; le propionate de vinyle ; l'alphaméthylstyrène ; le tertio-butylstyrène ; le butadiène ; le cyclohexadiène ; l'éthylène ; le propylène ; le vinyltoluène ; les esters d'acide acrylique ou méthacrylique et de 1,1-dihydroperfluoroalcanol ou de ses homologues ; les esters d'acide acrylique ou méthacrylique et de omégahydrydofluoroalcanol ; les esters d'acide acrylique ou méthacrylique et de fluoroalkylsulfoamido-alcool ; les esters d'acide acrylique ou méthacrylique et d'alcools fluoroalkylique ; les esters d'acide acrylique ou méthacrylique et de fluoroéthers d'alcools ; ou leurs mélanges.

5. Composition selon la revendication 4, **caractérisée par le fait que** les monoméres lipophiles (A) sont choisis dans le groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, le 2-(N-méthyl perfluorooctane sulfonamido)-éthylacrylate; le 2-(butylperfluorooctane sulfonamido)-éthylacrylate.

6. Composition selon l'une quelconque des revendications 3 à 5, **caractérisée par le fait que** les monomères polaires (B) sont choisis dans le groupe constitué par l'acide acrylique, l'acide méthacrylique, le N,N-diméthylacrylamide, le méthacrylate de diméthylaminoéthyle, le méthacrylate de diméthylaminoéthyle quatemisé, le(méth)acrylamide, le N-t-butylacrylamide, l'acide maleique, l'anhydride maléique et leurs demi-esters, les (méth)acrylates hydroxyalkylés, le chlorure de diallyldiméthylammonium, la vinylpyrrolidone, les éthers de vinyle, les maléimides, la vinylpyridine, le vinylimidazole, les composés polaires vinyliques hétérocycliques, le styrène sulfonate, l'alcool allylique, l'alcool vinylique, le vinylcaprolactame ou leurs mélanges.

7. Composition selon la revendication 6, **caractérisée par le fait que** les monomères polaires (B) sont choisis dans le groupe constitué par l'acide acrylique, le N,N-diméthylacrylamide, le méthacrylate de diméthylaminoéthyle, le méthacrylate de diméthylaminoéthyle quatemisé, la vinylpyrrolidone, et leurs mélanges.

8. Composition selon l'une quelconque des revendications 3 à 7, **caractérisée par le fait que** le macromère polysiloxane (C) correspond à la formule générale suivante (II) : 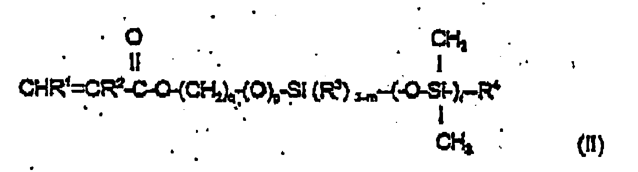 dans laquelle :
R¹ est hydrogène ou -COOH ;
R² est hydrogène, méthyle ou -CH₂COOH ;
R³ est alkyle, alcoxy ou alkylamino en C₁-C₆, aryle en C₆-C₁₂ ou hydroxyle ;
R⁴ est alkyle, alcoxy ou alkylamino en C₁-C₆, aryle en C₆-C₁₂ ou hydroxyle ;
q est un entier de 2 à 6 ;
p est 0 ou 1 ;
r est un nombre entier de 5 à 700 ;
m est un entier allant de 1 à 3 ;

9. Composition selon l'une quelconque des revendications 3 à 8, **caractérisée par le fait que** le macromère polysiloxane (C) correspond à la formule générale suivante : 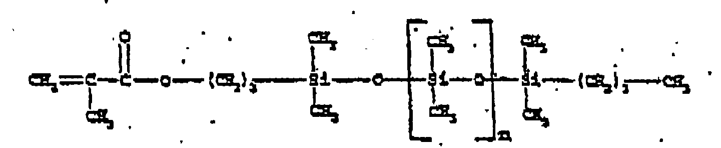 avec n étant un nombre allant de 5 à 700.

10. Composition selon l'une quelconque des revendications 1 à 9, **caractérisée par le fait qu'**elle contient au moins un polymère siliconé greffé susceptible d'être obtenu par polymérisation radicalaire à partir du mélange de monomères constitué par :
a) 60% en poids d'acrylate de tertiobutyle ;
b) 20% en poids d'acide acrylique ;
c) 20% en poids de macromère siliconé de formule : 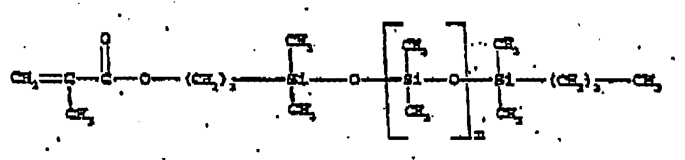 avec n étant un nombre allant de 5 à 700 ; les pourcentages en poids étant calculés par rapport au poids total des monomères.

11. Composition selon l'une quelconque des revendications 1 à 9, **caractérisée par le fait qu'**elle contient au moins un polymère siliconé greffé susceptible d'être obtenu par polymérisation radicalaire à partir du mélange de monomères constitué par :
a) 80% en poids d'acrylate de tertiobutyle ;
b) 20% en poids de macromère siliconé de formule : 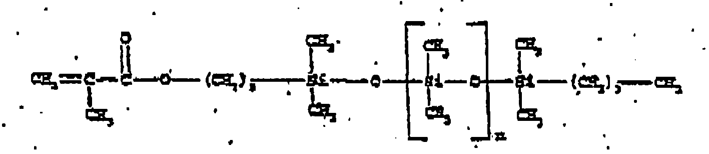 avec n étant un nombre allant de 5 à 700 ; les pourcentages en poids étant calculés par rapport au poids total des monomères.

12. Composition selon l'une quelconque des revendications 3 à 11, **caractérisée par le fait que** le polymère à squelette organique non-siliconé greffé par des monomères contenant un polysiloxane a un poids moléculaire moyen en nombre allant de 10.000 à 2.000.000 et une température de transition vitreuse Tg ou une température de fusion cristalline Tm d'au moins -20°C.

13. Composition selon l'une quelconque des revendications 1 ou 2, **caractérisée par le fait qu'**elle contient au moins un polymère à squelette organique non-siliconé greffé par des monomères contenant un polysiloxane, susceptible d'être obtenu par extrusion réactive d'un macromère polysiloxane ayant une fonction réactive terminale sur un polymère du type polyoléfine comportant des groupes réactifs susceptibles de réagir avec la fonction réactive terminale du macromère polysiloxane pour former une liaison covalente permettant le greffage de la silicone sur la chaîne principale de la polyoléfine.

14. Composition selon la revendication 13, **caractérisée par le fait que** la polyoléfine réactive est choisie dans le groupe constitué par les polyéthylènes ou les polymères de monomères dérivés de l'éthylène comportant des fonctions réactives susceptibles de réagir avec la fonction terminale du macromère polysiloxane.

15. Composition selon la revendication 13 ou 14, **caractérisée par le fait que** la polyoléfine réactive est choisie dans le groupe constitué par les copolymères d'éthylène ou de dérivés d'éthylène et de monomères choisis parmi ceux comportant une fonction carboxylique ; ceux comportant une fonction anhydride d'acide ; ceux comportant une fonction chlorure d'acide ; ceux comportant une fonction ester ; ceux comportant une fonction isocyanate.

16. Composition selon l'une quelconque des revendications 13 à 15, **caractérisée par le fait que** le macromère polysiloxane est un polysiloxane comportant un groupe fonctionnalisé, en bout de la chaîne polysiloxanique ou à proximité de l'extrémité de ladite chaîne, choisi dans le groupe constitué par les alcools, les thiols, les époxy, les amines primaires et secondaires.

17. Composition selon l'une quelconque des revendications 13 à 15, **caractérisée par le fait que** le macromère polysiloxane est un polysiloxane répondant à la formule générale (III) :
T-(CH₂)ₛ-Si-[-(OSiR⁵R⁶)ₜ-R⁷ ] _{y} (III)
dans laquelle T est choisi dans le groupe constitué par NH2, NHR', une fonction époxy, OH, SH ; R⁵, R⁶, R⁷ et R', indépendamment, désignent un alkyle en C₁-C₆, phényle, benzyle, ou alkylphényle en C₆-C₁₂, hydrogène ; s est un nombre de 2 à 100 ; t est un nombre de 0 à 1000 et y est un nombre de 1 à 3.

18. Composition selon la revendication 1, **caractérisée par le fait qu'**elle contient au moins un polymère siliconé greffé, à squelette polysiloxanique greffé par des monomères organiques non-siliconés, comprenant une chaîne principale de polysiloxane sur laquelle se trouve greffé, à l'intérieur de ladite chaîne ainsi qu'éventuellement à l'une au moins de ses extrémités, au moins un groupement organique ne comportant pas de silicone.

19. Composition selon la revendication 18, **caractérisée par le fait que** le polymère à squelette polysiloxanique greffé par des monomères organiques non-siliconés est susceptible d'être obtenu par copolymérisation radicalaire entre d'une part au moins un monomère organique anionique non-siliconé présentant une insaturation éthylénique et/ou un monomère organique hydrophobe non-siliconé présentant une insaturation éthylénique et d'autre part un polysiloxane présentant dans sa chaîne au moins un groupement fonctionnel capable de venir réagir sur lesdites insaturations éthyléniques desdits monoméres non-siliconés.

20. Composition selon la revendication 19, **caractérisée par le fait que** le monomère organique anionique à insaturation éthylénique est choisi, seul ou sous forme de mélange de monomères, parmi les acides carboxyliques insaturés, linéaires ou ramifiés.

21. Composition selon la revendication 20, **caractérisée par le fait que** le monomères organique anionique à insaturation éthylénique est choisi, seul ou sous forme de mélange de monomères, parmi l'acide acrylique, l'acide méthacrylique, l'acide maléique, l'anhydride maléique, l'acide itaconique, l'acide fumarique et l'acide crotonique ou leurs sels d'alcalins, d'alcalino-terreux ou d'ammonium, ou leurs mélanges.

22. Composition selon la revendication 11, **caractérisée par le fait que** le monomère organique hydrophobe à insaturation éthylénique est choisi, seul ou en mélange de monomères, parmi les esters d'acide acrylique d'alcanol et/ou les esters d'acide méthacrylique d'alcanol, de préférence l'alcanol étant en C₁-C₁₈.

23. Composition selon la revendication 22, **caractérisée par le fait que** le monomère organique hydrophobe à insaturation éthylénique est choisi, seul ou en mélanges de monomères dans le groupe constitué par le (méth)acrylate d'isooctyle, le (méth)acrylate d'isononyle, le 2-éthylhexyl(méth)acrylate, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle.

24. Composition selon l'une quelconque des revendications 18 à 23, **caractérisée par le fait que** le polymère siliconé greffé comprend sur la chaîne silicone principale, au moins un groupement organique à caractère anionique obtenu par l'(homo)polymérisation radicalaire d'au moins un monomère anionique de type acide carboxylique insaturé, partiellement ou totalement neutralisé sous la forme d'un sel.

25. Composition selon l'une quelconque des revendications 18 à 24, **caractérisée par le fait que** le polymère siliconé greffé est choisi parmi les polymères siliconés comportant dans leur structure le motif de formule (IV) suivant : 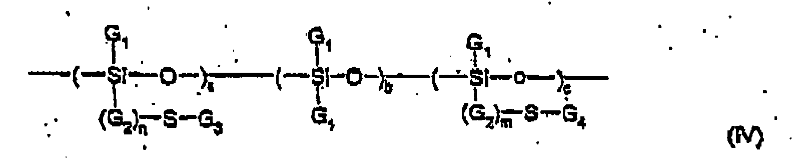 dans lequel les radicaux G₁, identiques ou différents, représentent l'hydrogène ou un radical alkyle en C₁-C₁₀ ou encore un radical phényle ; les radicaux G₂, identiques ou différents, représentent représente un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

26. Composition selon la revendication 25, **caractérisée par le fait que** le motif de formule (IV) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle (C₁-C₁₀).

27. Composition selon la revendication 25 ou 26, **caractérisée par le fait que** le motif de formule (IV) présente simultanément les caractéristiques suivantes :
- les radicaux G₁ désignent un radical méthyle ;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins un monomère du type (méth)acrylate d'isobuyle ou de méthyle.

28. Composition selon l'une quelconque des revendications 18 à 27, **caractérisée par le fait que** la masse moléculaire en nombre du polymère à squelette polysiloxanique greffé par des monomères organiques non-siliconés varie de 10 000 à 1 000 000 environ, et encore plus préférentiellement de 10 000 à 100 000 environ.

29. Composition selon l'une quelconque des revendications précédentes, **caractérisée par le fait que** ledit polymère cationique est choisi parmi les dérivés d'éther de cellulose quaternaires, les cyclopolymères, les polysaccharides cationiques et leurs mélanges.

30. Composition selon la revendication 29, **caractérisée par le fait que** ledit cyclopolymère est choisi parmi les copolymères du chlorure de diméthyldiallylammonium et d'acrylamide.

31. Composition selon la revendication 29, **caractérisée par le fait que** lesdits dérivés d'éther de cellulose quaternaires sont choisis parmi les hydroxyéthylcelluloses ayant réagi avec un époxyde substitué par un groupement triméthylammonium.

32. Composition selon la revendication 29, **caractérisée par le fait que** lesdits polysaccharides cationiques sont choisis parmi les gommes de guar modifiées par un sel de 2,3-époxypropyl triméthylammonium.

33. Composition selon l'une quelconque des revendications précédentes,
**caractérisée par le fait que** la silicone aminée est choisie parmi :
(a) les polysiloxanes dénommés dans le dictionnaire CTFA "amodiméthicone" et répondant à la formule: 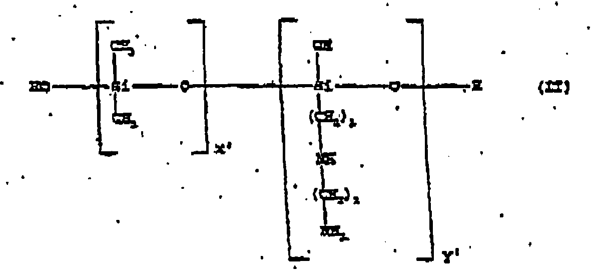 dans laquelle x' et y' sont des nombres entiers dépendant du poids moléculaire, généralement tels que ledit poids moléculaire en nombre est compris entre 5 000 et 500 000 ;
(b) les polymères cationiques siliconés répondant à la formule :
R'ₐG₃₋ₐ-Si(OSiG₂)ₙ-(OSiG_{b}R'_{2-b})ₘ-O-SiG₃₋ₐ-R'ₐ (III)
dans laquelle :
G est un atome d'hydrogène, ou un groupement phényle, OH, ou alkyle en C₁-C₈, par exemple méthyle,
a désigne le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R' est un radical monovalent de formule -C_{q}H_{2q}L dans laquelle q est un nombre de 2 à 8 et L est un groupement aminé éventuellement quatemisé choisi parmi les groupements :
- NR"-CH₂-CH₂-N'(R")₂
- N(R")₂
- N^{⊕}(R")₃A⁻
- N^{⊕}(R")₃A⁻
- N^{⊕}(R")₃A⁻
- N(R")-CH₂-CH₂-N^{⊕}R" H₂ A⁻,
dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
(c) les polymères cationiques siliconés répondant à la formule : 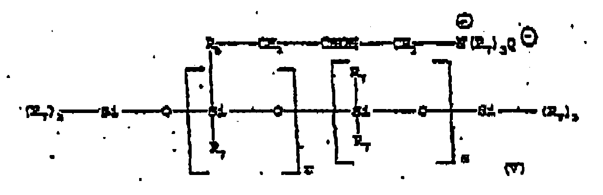 dans laquelle :
R₇ représente un radical hydrocarboné monovalent ayant de 1 à 18 atomes de carbone, et en particulier un radical alkyle en C₁-C₁₈, ou alcényle en C₂-C₁₈, par exemple méthyle ;
R₈ représente un radical hydrocarboné divalent, noatmment un radical alkylène en C₁-C₁₈ ou un radical alkylèneoxy divalent en C₁-C₁₈, par exemple en C₁-C₈ ;
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

34. Composition selon l'une quelconques des revendications précédentes,
**caractérisée par le fait que** ladite base lavante est présente à une teneur pondérale comprise entre 4 % et 50 % par rapport au poids total de la composition.

35. Composition selon la revendication 34, **caractérisée par le fait que** ladite teneur est comprise entre 10 % et 35 %.

36. Composition selon la revendication 35, **caractérisée par le fait que** ladite teneur est comprise entre 12 % à 25 %.

37. Composition selon l'une quelconque des revendications précédentes,
**caractérisée par le fait que** ledit polymère cationique est présent à une teneur pondérale comprise entre 0,001 % et 10 % par rapport au poids total de la composition.

38. Composition selon la revendication 37, **caractérisée par le fait que** ladite teneur est comprise entre 0,005 % et 5 %.

39. Composition selon la revendication 38, **caractérisée par le fait que** ladite teneur est comprise entre 0,01 % et 3 %.

40. Composition selon l'une quelconque des revendications précédentes,
**caractérisée par le fait que** ledit polymère siliconé greffé est présent à une teneur pondérale comprise entre 0,01 % et 20 % par rapport au poids total de la composition.

41. Composition selon la revendication 40, **caractérisée par le fait que** ladite teneur est comprise entre 0,1 % et 15 %.

42. Composition selon la revendication 41, **caractérisée par le fait que** ladite teneur est comprise entre 0,5 % et 10%.

43. Composition selon l'une quelconque des revendications précédentes,
**caractérisée par le fait que** ladite silicone aminée est présente à une teneur pondérale comprise entre 0,05 et 10% par rapport au poids total de la composition.

44. Composition selon la revendication 43, **caractérisée par le fait que** ladite teneur est comprise entre 0,1 et 5 % par rapport au poids total de la composition.

45. Composition selon l'une quelconque des revendications précédentes,
**caractérisée par le fait qu'**elles présentent un pH compris entre 3 et 10.

46. Utilisation d'une composition telle définie à l'une quelconque des revendications précédentes pour le nettoyage et/ou le coiffage et/ou le conditionnement des cheveux.

47. Utilisation d'une silicone aminée pour améliorer l'effet coiffant d'une composition capillaire détergente contenant au moins un polymère cationique et au moins un polymère siliconé greffé tel que défini à l'une des revendications 1 à 28.

48. Procédé de lavage et de conditionnement des fibres kératiniques telles que les cheveux consistant à appliquer sur lesdites fibres mouillées une quantité efficace d'une composition telle que définie à l'une quelconque des revendications 1 à 45, puis à effectuer un rinçage à l'eau après un éventuel temps de pause.

## Claims

1. Detergent and conditioning hair composition, **characterized in that** it comprises, in a cosmetically acceptable medium, (A) a washing base comprising one or more surfactants chosen from anionic, amphoteric, nonionic, zwitterionic, cationic and their mixtures and (B) a conditioning system comprising at least one cationic polymer and a mixture of at least one aminosilicone and of at least one grafted silicone polymer comprising a polysiloxane portion and a portion consisting of a non-silicone organic chain, one of the two portions constituting the main chain of the polymer, the other being grafted onto the said main chain, the grafted silicone polymers being chosen from polymers having a non-silicone organic skeleton, consisting of an organic main chain formed from organic monomers containing no silicone, on which is grafted, inside the said chain and optionally on at least one of its ends, at least one polysiloxane macromer, or polymers with a polysiloxane skeleton grafted with non-silicone monomers, which may be obtained by radical copolymerization between, on the one hand, at least one ethylenically unsaturated non-silicone anionic organic monomer and/or an ethylenically unsaturated non-silicone hydrophobic organic monomer, and, on the other hand, a silicone containing in its chain at least one functional group capable of reacting with the said ethylenic unsaturations of the said non-silicone monomers, forming a covalent bond.

2. Composition according to Claim 1, **characterized in that** the non-silicone organic monomers constituting the main chain of the grafted silicone polymer are chosen from the group consisting of monomers containing ethylenic unsaturation which are polymerizable via a radical route, monomers which are polymerizable by polycondensation and monomers which involve ring opening.

3. Composition according to either one of Claims 1 to 2, **characterized in that** it contains at least one grafted silicone polymer comprising:
a) from 0 to 98% by weight of at least one lipophilic monomer (A) of low polarity containing ethylenic unsaturation, which is polymerizable via a radical route;
b) from 0 to 98% by weight of at least one polar hydrophilic monomer (B) containing ethylenic unsaturation, which is copolymerizable with the (A)-type monomer(s);
c) from 0.01 to 50% by weight of at least one polysiloxane macromer (C) of general formula:
X(Y)ₙSi(R)₃₋ₘ Zₘ (I)
where:
X denotes a vinyl group which is copolymerizable with the monomers (A) and (B);
Y denotes a divalent bonding group;
R denotes a hydrogen, a C₁-C₆ alkyl or alkoxy or a C₆-C₁₂ aryl;
Z denotes a monovalent polysiloxane unit having a number-average molecular weight of at least 500;
n is 0 or 1 and m is an integer ranging from 1 to 3; the percentages being calculated relative to the total weight of the monomers (A), (B) and (C).

4. Composition according to Claim 3, **characterized in that** the lipophilic monomers (A) are chosen from the group consisting of acrylic or methacrylic acid esters of C₁-C₁₈ alcohols; styrene; polystyrene macromers; vinyl acetate; vinyl propionate; α-methylstyrene; tert-butylstyrene; butadiene; cyclohexadiene; ethylene; propylene; vinyltoluene; acrylic or methacrylic acid esters of 1,1-dihydroperfluoroalkanol or of homologues thereof; acrylic or methacrylic acid esters of ω-hydridofluoroalkanol; acrylic or methacrylic acid esters of fluoroalkylsulphoamido alcohol; acrylic or methacrylic acid esters of fluoroalkyl alcohols; acrylic or methacrylic acid esters of fluoroether alcohols; or mixtures thereof.

5. Composition according to Claim 4, **characterized in that** the lipophilic·monomers (A) are chosen from the group consisting of n-butyl methacrylate, isobutyl methacrylate, tert-butyl acrylate, tert-butyl methacrylate, 2-ethylhexyl methacrylate, methyl methacrylate, 2-(N-methyl-perfluorooctanesulphonamido)ethyl acrylate and 2-(butylperfluorooctanesulphonamido)ethyl acrylate.

6. Composition according to any one of Claims 3 to 5, **characterized in that** the polar monomers (B) are chosen from the group consisting of acrylic acid, methacrylic acid, N,N-dimethylacrylamide, dimethylaminoethyl methacrylate, quaternized dimethylaminoethyl methacrylate, (meth)acrylamide, N-t-butylacrylamide, maleic acid, maleic anhydride and semiesters thereof, hydroxyalkyl (meth)acrylates, diallyldimethylammonium chloride, vinylpyrrolidone, vinyl ethers, maleimides, vinylpyridine, vinylimidazole, heterocyclic vinyl polar compounds, styrene sulphonate, allyl alcohol, vinyl alcohol and vinyl caprolactam, or mixtures thereof.

7. Composition according to Claim 6, **characterized in that** the polar monomers (B) are chosen from the group consisting of acrylic acid, N,N-dimethylacrylamide, dimethylaminoethyl methacrylate, quaternized dimethylaminoethyl methacrylate and vinylpyrrolidone, and mixtures thereof.

8. Composition according to any one of Claims 3 to 7, **characterized in that** the polysiloxane macromer (C) corresponds to the general formula (II) below: 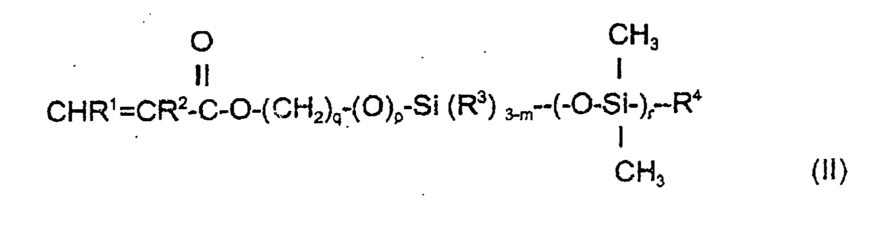 in which:
R¹ is hydrogen or -COOH;
R² is hydrogen, methyl or -CH₂COOH;
R³ is C₁-C₆ alkyl, alkoxy, or alkylamino, C₆-C₁₂ aryl or hydroxyl;
R⁴ is C₁-C₆ alkyl, alkoxy or alkylamino, C₆-C₁₂ aryl or hydroxyl;
q is an integer from 2 to 6;
p is 0 or 1;
r is an integer from 5 to 700;
m is an integer ranging from 1 to 3.

9. Composition according to any one of Claims 3 to 8, **characterized in that** the polysiloxane macromer (C) corresponds to the general formula below: 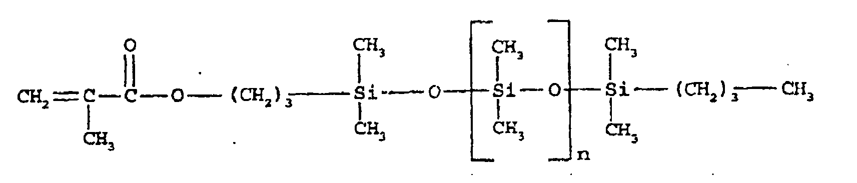 with n being a number ranging from 5 to 700.

10. Composition according to any one of Claims 1 to 9, **characterized in that** it contains at least one grafted silicone polymer which can be obtained by radical polymerization starting with the monomer mixture consisting of:
a) 60% by weight of tert-butyl acrylate;
b) 20% by weight of acrylic acid;
c) 20% by weight of silicone macromer of formula: 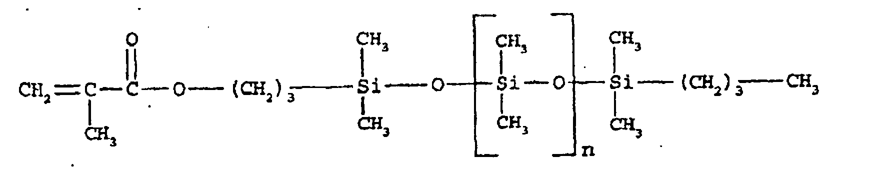 with n being a number ranging from 5 to 700; the weight percentages being calculated relative to the total weight of the monomers.

11. Composition according to any one of Claims 1 to 9, **characterized in that** it contains at least one grafted silicone polymer which can be obtained by radical polymerization starting with the monomer mixture consisting of:
a) 80% by weight of tert-butyl acrylate;
b) 20% by weight of silicone macromer of formula: 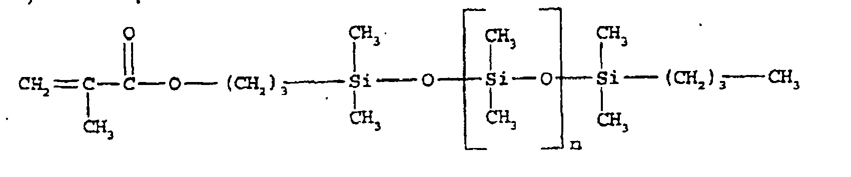 with n being a number ranging from 5 to 700; the weight percentages being calculated relative to the total weight of the monomers.

12. Composition according to any one of Claims 3 to 11, **characterized in that** the polymer containing a non-silicone organic skeleton grafted with monomers containing a polysiloxane has a number-average molecular weight ranging from 10,000 to 2,000,000 and a glass transition temperature Tg or a crystalline melting point Tm of at least -20°C.

13. Composition according to either one of Claims 1 and 2, **characterized in that** it contains at least one polymer containing a non-silicone organic skeleton grafted with monomers containing a polysiloxane, which can be obtained by reactive extrusion of a polysiloxane macromer having a terminal reactive function, on a polyolefin-type polymer containing reactive groups which can react with the reactive terminal function of the polysiloxane macromer in order to form a covalent bond allowing grafting of the silicone to the main chain of the polyolefin.

14. Composition according to Claim 13, **characterized in that** the reactive polyolefin is chosen from the group consisting of polyethylenes or polymers of ethylene-derived monomers containing reactive functions which can react with the terminal function of the polysiloxane macromer.

15. Composition according to Claim 13 or 14, **characterized in that** the reactive polyolefin is chosen from the group consisting of copolymers of ethylene or of ethylene derivatives and of monomers chosen from those containing a carboxylic function; those containing an acid anhydride function; those containing an acid chloride function; those containing an ester function; those containing an isocyanate function.

16. Composition according to any one of Claims 13 to 15, **characterized in that** the polysiloxane macromer is a polysiloxane containing a functionalized group, at the end of the polysiloxane chain or close to the end of the said chain, chosen from the group consisting of alcohols, thiols, epoxy groups and primary and secondary amines.

17. Composition according to any one of Claims 13 to 15, **characterized in that** the polysiloxane macromer is a polysiloxane corresponding to the general formula (III) :
T-(CH₂)ₛ-Si-[-(O-SiR⁵R⁶)ₜ-R⁷]_{y} (III)
in which T is chosen from the group consisting of NH₂, NHR', an epoxy, OH, or SH function; R⁵, R⁶, R⁷ and R', independently denote a C₁-C₆ alkyl, phenyl, benzyl, C₆-C₁₂ alkylphenyl or hydrogen; s is a number from 2 to 100; t is a number from 0 to 1000 and y is a number from 1 to 3.

18. Composition according to Claim 1, **characterized in that** it contains at least one grafted silicone polymer, containing a polysiloxane skeleton grafted with non-silicone organic monomers, comprising a polysiloxane main chain on which is grafted, inside the said chain and optionally on at least one of its ends, at least one organic group containing no silicone.

19. Composition according to Claim 18, **characterized in that** the polymer containing a polysiloxane skeleton grafted with non-silicone organic monomers can be obtained by radical copolymerization between, on the one hand, at least one non-silicone anionic organic monomer containing ethylenic unsaturation and/or a non-silicone hydrophobic organic monomer containing ethylenic unsaturation, and, on the other hand, a polysiloxane containing in its chain at least one functional group capable of reacting with the said ethylenic unsaturations of the said non-silicone monomers.

20. Composition according to Claim 19, **characterized in that** the anionic organic monomer containing ethylenic unsaturation is chosen, alone or in the form of a mixture of monomers, from linear or branched, unsaturated carboxylic acids.

21. Composition according to Claim 20, **characterized in that** the anionic organic monomer containing ethylenic unsaturation is chosen, alone or in the form of a mixture of monomers, from acrylic acid, methacrylic acid, maleic acid, maleic anhydride, itaconic acid, fumaric acid and crotonic acid, or the alkali metal, alkaline-earth metal or ammonium salts thereof, or mixtures thereof.

22. Composition according to Claim 19, **characterized in that** the hydrophobic organic monomer containing ethylenic unsaturation is chosen, alone or as a mixture of monomers, from acrylic acid esters of an alkanol and/or methacrylic acid esters of an alkanol, the alkanol preferably being C₁-C₁₈.

23. Composition according to Claim 22, **characterized in that** the hydrophobic organic monomer containing ethylenic unsaturation is chosen, alone or as a mixture of monomers, from the group consisting of isooctyl (meth)acrylate, isononyl (meth)acrylate, 2-ethylhexyl (meth)acrylate, lauryl (meth)acrylate, isopentyl (meth)acrylate, n-butyl (meth)acrylate, isobutyl (meth)acrylate, methyl (meth)acrylate, tert-butyl (meth)acrylate, tridecyl (meth)acrylate and stearyl (meth)acrylate.

24. Composition according to any one of Claims 18 to 23, **characterized in that** the grafted silicone polymer comprises, on the main silicone chain, at least one organic group of anionic nature obtained by radical (homo)polymerization of at least one anionic monomer of unsaturated carboxylic acid type, partially or totally neutralized in the form of a salt.

25. Composition according to any one of Claims 18 to 24, **characterized in that** the grafted silicone polymer is chosen from silicone polymers containing in their structure the unit of formula (IV) below: 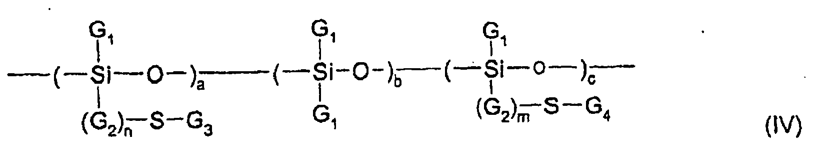 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymer residue resulting from the (homo)polymerization of at least one anionic monomer containing ethylenic unsaturation; G₄ represents a polymer residue resulting from the (homo)polymerization of at least one hydrophobic monomer containing ethylenic unsaturation; m and n are equal to 0 or 1; a is an integer ranging from 0 to 50; b is an integer which may be between 10 and 350, c is an integer ranging from 0 to 50; with the proviso that one of the parameters a and c is other than 0.

26. Composition according to Claim 25, **characterized in that** the unit of formula (IV) has at least one of the following characteristics:
- the radicals G₁ denote a C₁-C₁₀ alkyl radical;
- n is non-zero, and the radicals G₂ represent a divalent C₁-C₃ radical;
- G₃ represents a polymer radical resulting from the (homo)polymerization of at least one monomer of the carboxylic acid type containing ethylenic unsaturation;
- G₄ represents a polymer radical resulting from the (homo)polymerization of at least one monomer of the (C₁-C₁₀)alkyl (meth)acrylate type.

27. Composition according to Claim 25 or 26, **characterized in that** the unit of formula (IV) simultaneously has the following characteristics:
- the radicals G₁ denote a methyl radical;
- n is non-zero, and the radicals G₂ represent a propylene radical;
- G₃ represents a polymer radical resulting from the (homo)polymerization of at least acrylic acid and/or methacrylic acid;
- G₄ represents a polymer radical resulting from the (homo)polymerization of at least one monomer of the isobutyl or methyl (meth)acrylate type.

28. Composition according to any one of Claims 18 to 27, **characterized in that** the number-average molecular mass of the polymer containing a polysiloxane skeleton grafted with non-silicone organic monomers ranges from 10,000 to 1,000,000 approximately and even more preferably from 10,000 to 100,000 approximately.

29. Composition according to any one of the preceding claims, **characterized in that** the said cationic polymer is chosen from quaternary cellulose ether derivatives, cyclopolymers and cationic polysaccharides, and mixtures thereof.

30. Composition according to Claim 29, **characterized in that** the said cyclopolymer is chosen from copolymers of dimethyldiallylammonium chloride and of acrylamide. ,

31. Composition according to Claim 29, **characterized in that** the said quaternary cellulose ether derivatives are chosen from hydroxyethylcelluloses which have reacted with an epoxide substituted with a trimethylammonium group.

32. Composition according to Claim 29, **characterized in that** the said cationic polysaccharides are chosen from guar gums modified with a 2,3-epoxypropyltrimethylammonium salt.

33. Composition according to any one of the preceding claims, **characterized in that** the aminosilicone is chosen from:
(a) the polysiloxanes referred to in the CTFA dictionary as "amodimethicone" and corresponding to the formula: 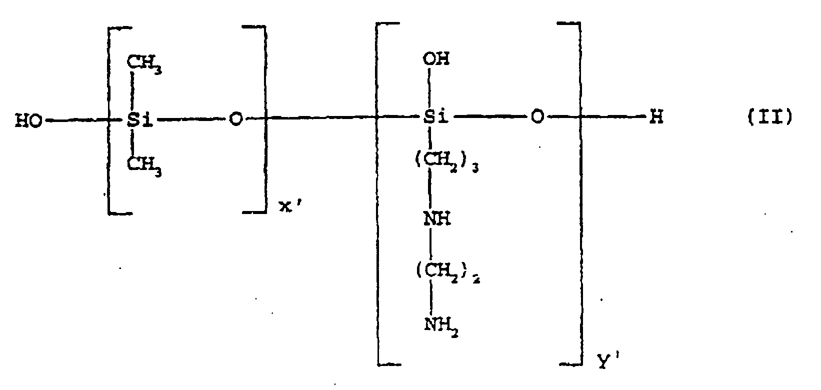 in which x' and y' are integers dependent on the molecular weight, generally such that the said number-average molecular weight is between 5000 and 500,000;
(b) cationic silicone polymers corresponding to the formula:
R'ₐG₃₋ₐ-Si(OSiG₂)ₙ-(OSiG_{b}R'_{2-b})ₘ-O-SiG₃₋ₐ-R'ₐ (III)
in which:
G is a hydrogen atom or a phenyl, OH or C₁-C₈ alkyl, for example methyl, group,
a denotes the number 0 or an integer from 1 to 3, in particular 0,
b denotes 0 or 1, and in particular 1,
m and n are numbers such that the sum (n + m) can range especially from 1 to 2000 and in particular from 50 to 150, it being possible for n to denote a number from 0 to 1999 and in particular from 49 to 149 and it being possible for m to denote a number from 1 to 2000, and in particular from 1 to 10;
R' is a monovalent radical of formula -C_{q}H_{2q}L in which q is a number from 2 to 8 and L is an optionally quaternized amine group chosen from the groups:
- NR"-CH₂-CH₂-N'(R")₂
- N(R")₂
- N^{⊕}(R")₃A⁻
- N^{⊕}(R")₃A⁻
- N^{⊕}(R")₃A⁻
- N(R")-CH₂-CH₂-N^{⊕}R"H₂A⁻,
in which R" can denote hydrogen, phenyl, benzyl or a saturated monovalent hydrocarbon-based radical, for example an alkyl radical containing from 1 to 20 carbon atoms, and A⁻ represents a halide ion such as, for example, fluoride, chloride, bromide or iodide;
(c) cationic silicone polymers corresponding to the formula: 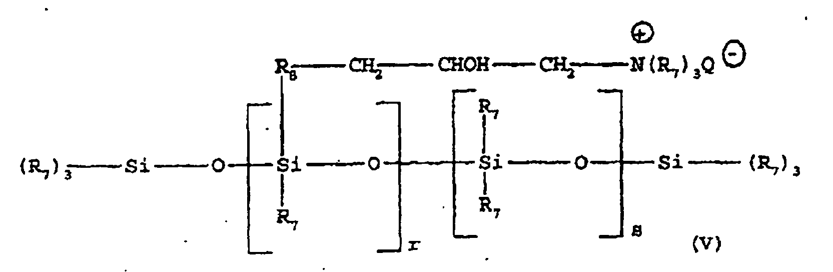 in which:
R⁷ represents a monovalent hydrocarbon-based radical having from 1 to 18 carbon atoms, and in particular a C₁-C₁₈ alkyl or C₂-C₁₈ alkenyl radical, for example methyl;
R₈ represents a divalent hydrocarbon-based radical, in particular a C₁-C₁₈ alkylene radical or a divalent C₁-C₁₈, for example C₁-C₈, alkylenoxy radical;
Q⁻ is a halide ion, in particular chloride;
r represents an average statistical value from 2 to 20 and in particular from 2 to 8;
s represents an average statistical value from 20 to 200 and in particular from 20 to 50.

34. Composition according to any one of the preceding claims, **characterized in that** the said washing base is present at a weight content of between 4% and 50% relative to the total weight of the composition.

35. Composition according to Claim 34, **characterized in that** the said content is between 10% and 35%.

36. Composition according to Claim 35, **characterized in that** the said content is between 12% and 25%.

37. Composition according to any one of the preceding claims, **characterized in that** the said cationic polymer is present at a weight content of between 0.001% and 10% relative to the total weight of the composition.

38. Composition according to Claim 37, **characterized in that** the said content is between 0.005% and 5%.

39. Composition according to Claim 38, **characterized in that** the said content is between 0.01% and 3%.

40. Composition according to any one of the preceding claims, **characterized in that** the said grafted silicone polymer is present at a weight content of between 0.01% and 20% relative to the total weight of the composition.

41. Composition according to Claim 40, **characterized in that** the said content is between 0.1% and 15%.

42. Composition according to Claim 41, **characterized in that** the said content is between 0.5% and 10%.

43. Composition according to any one of the preceding claims, **characterized in that** the said aminosilicone is present at a weight content of between 0.05 and 10% relative to the total weight of the composition.

44. Composition according to Claim 43, **characterized in that** the said content is between 0.1 and 5% relative to the total weight of the composition.

45. Composition according to any one of the preceding claims, **characterized in that** they have a pH of between 3 and 10.

46. Use of a composition as defined in any one of the preceding claims, for cleaning and/or styling and/or conditioning the hair.

47. Use of an aminosilicone for improving the styling effect of a detergent hair composition containing at least one cationic polymer and at least one grafted silicone polymer as defined in any one of Claims 1 to 28.

48. Process for washing and conditioning keratin fibres such as the hair, which consists in applying an effective amount of a composition as defined in any one of Claims 1 to 45 to the said wet fibres, and then in rinsing them with water, after optionally leaving the composition to stand on the fibres for a period of time.

## Patentansprüche

1. Reinigende und konditionierende Zusammensetzung für das Haar, **dadurch gekennzeichnet, dass** sie in einem kosmetisch akzeptablen Medium (A) eine reinigende Basisformulierung, die einen oder mehrere grenzflächenaktive Stoffe enthält, die unter den anionischen, amphoteren, nichtionischen, zwitterionischen oder kationischen grenzflächenaktiven Stoffen und deren Gemischen ausgewählt sind, und (B) ein Konditioniersystem enthält, das mindestens ein kationisches Polymer und ein Gemisch von mindestens einem aminierten Silicon und mindestens einem gepfropften Siliconpolymer mit einem Polysiloxanbereich und einem Bereich, der aus einer nicht siliconhaltigen, organischen Kette besteht, wobei ein Bereich die Hauptkette des Polymers bildet und der andere auf die Hauptkette gepfropft ist, enthält, wobei die gepfropften Siliconpolymere unter den Polymeren mit einem nicht siliconhaltigen organischen Grundgerüst, das aus einer organischen Hauptkette besteht, die aus organischen Monomeren gebildet ist, die kein Silicon enthalten, und auf die im Inneren der Kette sowie gegebenenfalls an mindestens einem ihrer Enden mindestens ein Polysiloxanmakromer gepfropft ist, oder den Polymeren mit einem mit nicht siliconhaltigen Monomeren gepfropften Polysiloxangerüst ausgewählt sind, die durch radikalische Copolymerisation einerseits mindestens eines nicht siliconhaltigen anionischen organischen Monomers, das eine ethylenische Doppelbindung aufweist, und/oder mindestens eines nicht siliconhaltigen hydrophoben organischen Monomers, das eine ethylenische Doppelbindung aufweist, und andererseits eines Silicons erhältlich ist, das in seiner Kette mindestens eine funktionelle Gruppe enthält, die befähigt ist, mit den ethylenisch ungesättigten Bindungen der nicht siliconierten Monomere unter Ausbildung einer kovalenten Bindung zu reagieren.

2. Zusammensetzung nach Anspruch 1, **dadurch gekennzeichnet, dass** die nicht siliconhaltigen, organischen Monomere, die die Hauptkette des gepfropften Siliconpolymers bilden, unter den auf radikalischem Wege polymerisierbaren Monomeren mit ethylenischer Doppelbindung, den durch Polykondensation polymerisierbaren Monomeren und den unter Ringöffnung polymerisierbaren Monomeren ausgewählt sind.

3. Zusammensetzung nach einem der Ansprüche 1 bis 2, **dadurch gekennzeichnet, dass** sie mindestens ein gepfropftes Siliconcopolymer enthält, das aufweist:
a) 0 bis 98 Gew.-% mindestens eines lipophilen Monomers (A) von geringer Polarität mit ethylenischer Doppelbindung, das radikalisch polymerisierbar ist;
b) 0 bis 98 Gew.-% mindestens eines polaren hydrophilen Monomers (B) mit ethylenischer Doppelbindung, das mit dem (den) Monomer(en) vom Typ (A) copolymerisierbar ist;
c) 0,01 bis 50 Gew.-% mindestens eines Polysiloxanmakromers (C) der allgemeinen Formel:
X(Y)ₙSi(R)₃₋ₘZₘ (I)
worin bedeuten:
X eine mit den Monomeren (A) und (B) copolymerisierbare Vinylgruppe;
Y eine zweiwertige Verbindungsgruppe;
R Wasserstoff, Alkyl oder Alkoxy mit 1 bis 6 Kohlenstoffatomen, C₆₋₁₂-Aryl;
Z eine einwertige Polysiloxaneinheit mit einem Zahlenmittel des Molekulargewichts von mindestens 500;
n 0 oder 1 und m eine ganze Zahl von 1 bis 3; wobei die Prozentzahlen bezogen auf das Gesamtgewicht der Monomeren (A), (B) und (C) angegeben sind.

4. Zusammensetzung nach Anspruch 3, **dadurch gekennzeichnet, dass** die lipophilen Monomere (A) unter Estern von Acrylsäure oder Methacrylsäure und C₁₋₁₈-Alkoholen; Styrol; Polystyrolmakromeren; Vinylacetat; Vinylpropionat; α-Methylstyrol; tert.-Butylstyrol; Butadien; Cyclohexadien; Ethylen; Propylen; Vinyltoluol; Estern von Acrylsäure oder Methacrylsäure und 1,1-Dihydroperfluoralkanolen oder ihren homologen Verbindungen; Estern von Acrylsäure oder Methacrylsäure und omega-Hydridofluoralkanolen; Estern von Acrylsäure oder Methacrylsäure und Fluoralkylsulfonamidoalkoholen; Estern von Acrylsäure oder Methacrylsäure und Fluoralkylalkoholen; Estern von Acrylsäure oder Methacrylsäure und Fluorethern von Alkoholen; oder deren Gemischen ausgewählt sind.

5. Zusammensetzung nach Anspruch 4, **dadurch gekennzeichnet, dass** die lipophilen Monomere (A) unter n-Butylmethacrylat, Isobutylmethacrylat, tert.-Butylacrylat, tert.-Butylmethacrylat, 2-Ethylhexyl-methacrylat, Methylmethacrylat, 2-(N-Methylperfluoroctan-sulfonamido)-ethylacrylat und 2-(N-Butylperfluoroctan-sulfon-amido)-ethylacrylat ausgewählt sind.

6. Zusammensetzung nach einem der Ansprüche 3 bis 5, **dadurch gekennzeichnet, dass** die polaren Monomere (B) unter Acrylsäure, Methacrylsäure, N,N-Dimethylacrylamid, Dimethylaminoethylmethacrylat, quaternisiertem Dimethylaminoethylmethacrylat, (Meth)acrylamid, N-t-Butyl-acrylamid, Maleinsäure, Maleinsäureanhydrid und seinen Halbestern, hydroxyalkylierten (Meth)acrylaten, Diallyldimethylammoniumchlorid, Vinylpyrrolidon, Vinylethern, Maleimiden, Vinylpyridin, Vinylimidazol, heterocyclischen polaren Vinylverbindungen, Styrolsulfonat, Allylalkohol, Vinylalkohol, Vinylcaprolactam oder deren Gemischen ausgewählt sind.

7. Zusammensetzung nach Anspruch 6, **dadurch gekennzeichnet, dass** die polaren Monomere (B) unter Acrylsäure, N,N-Dimethylacrylamid, Dimethylaminoethylmethacrylat, quaternisiertem Dimethylamino-ethylmethacrylat, Vinylpyrrolidon und deren Gemischen ausgewählt sind.

8. Zusammensetzung nach einem der Ansprüche 3 bis 7, **dadurch gekennzeichnet, dass** das Polysiloxanmakromer (C) der folgenden allgemeinen Formel (II) entspricht: 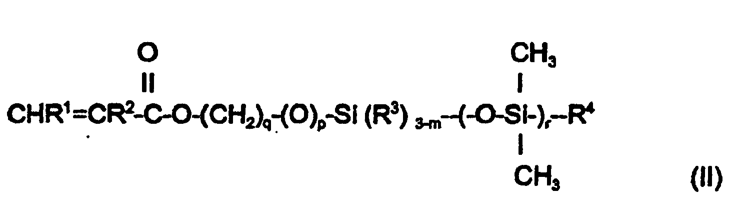 worin bedeuten:
R¹ Wasserstoff oder -COOH,
R² Wasserstoff, Methyl oder -CH₂COOH,
R³ Alkyl, Alkoxy oder Alkylamino mit 1 bis 6 Kohlenstoffatomen, C₆₋₁₂-Aryl oder Hydroxy,
R⁴ Alkyl, Alkoxy oder Alkylamino mit 1 bis 6 Kohlenstoffatomen, C₆₋₁₂-Aryl oder Hydroxy,
q eine ganze Zahl von 2 bis 6,
p 0 oder 1,
r eine ganze Zahl von 5 bis 700,
m eine ganze Zahl von 1 bis 3.

9. Zusammensetzung nach einem der Ansprüche 3 bis 8, **dadurch gekennzeichnet, dass** das Polysiloxanmakromer (C) der folgenden allgemeinen Formel entspricht: 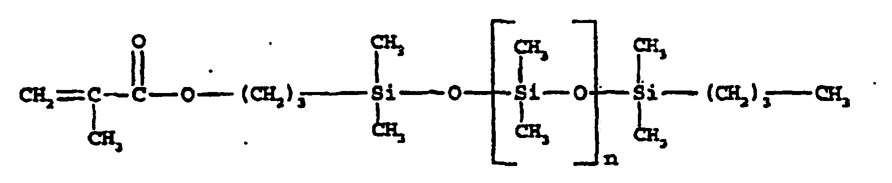 wobei n eine Zahl im Bereich von 5 bis 700 bedeutet.

10. Zusammensetzung nach einem der Ansprüche 1 bis 9, **dadurch gekennzeichnet, dass** sie mindestens ein gepfropftes Siliconpolymer enthält, das durch radikalische Polymerisation ausgehend von folgendem Monomerengemisch erhältlich ist:
60 Gew.-% *t*-Butylacrylat,
20 Gew.-% Acrylsäure,
20 Gew.-% Siliconmakromer der Formel: 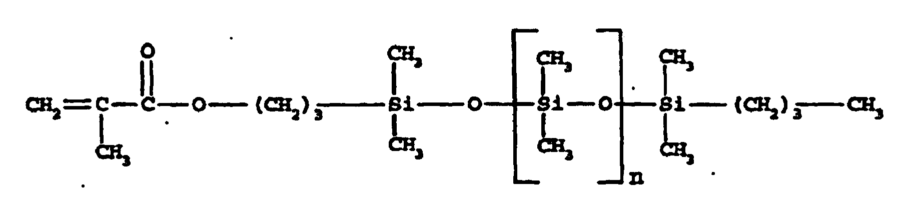 wobei n eine Zahl von 5 bis 700 ist; die Prozentzahlen sind bezogen auf das Gesamtgewicht der Monomere angegeben.

11. Zusammensetzung nach einem der Ansprüche 1 bis 9, **dadurch gekennzeichnet, dass** sie mindestens ein gepfropftes Siliconpolymer enthält, das durch radikalische Polymerisation ausgehend von folgendem Monomerengemisch erhältlich ist:
a) 80 Gew.-% *t*-Butylacrylat,
b) 20 Gew.-% Siliconmakromer der Formel: 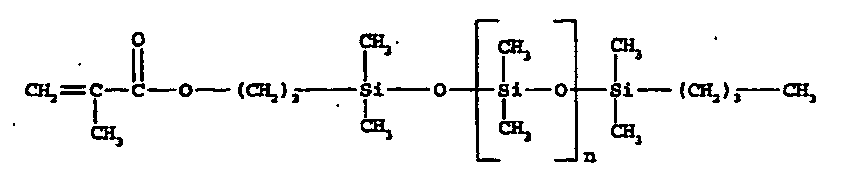 wobei n eine Zahl von 5 bis 700 ist; die Prozentzahlen sind bezogen auf das Gesamtgewicht der Monomere angegeben.

12. Zusammensetzung nach einem der Ansprüche 3 bis 11, **dadurch gekennzeichnet, dass** das Polymer mit einem nicht siliconhaltigen, organischen Grundgerüst, das mit polysiloxanhaltigen Monomeren gepfropft ist, ein Zahlenmittel des Molekulargewichts von 10 000 bis 2 000 000 und eine Glasübergangstemperatur Tg oder eine kristalline Schmelztemperatur Tₘ von mindestens -20 °C aufweist.

13. Zusammensetzung nach einem der Ansprüche 1 oder 2, **dadurch gekennzeichnet, dass** sie mindestens ein Polymer mit einem nicht siliconhaltigen, organischen Grundgerüst enthält, das mit polysiloxanhaltigen Monomeren gepfropft ist, das durch reaktive Extrusion eines Polysiloxanmakromers mit einer reaktiven Endgruppe und eines Polymers vom Polyolefintyp erhältlich ist, das Gruppen aufweist, die mit der reaktiven Endgruppe des Polysiloxanmakromers reagieren können, wodurch eine kovalente Bindung ausgebildet wird, über die das Silicon auf die Polyolefin-Hauptkette gepfropft werden kann.

14. Zusammensetzung nach Anspruch 13, **dadurch gekennzeichnet, dass** das reaktive Polyolefin unter den Polyethylenen oder den Polymeren von Monomeren, die von Ethylen abgeleitet sind, ausgewählt ist, die reaktive Gruppen aufweisen, die mit der Endgruppe des Polysiloxanmakromers reagieren können.

15. Zusammensetzung nach Anspruch 13 oder 14, **dadurch gekennzeichnet, dass** das reaktive Polyolefin unter den Copolymeren von Ethylen oder Derivaten von Ethylen und Monomeren, die eine Carboxygruppe aufweisen; Monomeren, die eine Anhydridgruppe aufweisen; Monomeren, die ein Säurechloridgruppe enthalten; Monomeren, die eine Estergruppe aufweisen; und Monomeren, die eine Isocyanatgruppe enthalten, ausgewählt ist.

16. Zusammensetzung nach einem der Ansprüche 13 bis 15, **dadurch gekennzeichnet, dass** das Polysiloxanmakromer ein Polysiloxan ist, das am Ende der Polysiloxankette oder in der Nähe des Kettenendes eine funktionelle Gruppe aufweist, die unter Alkoholen, Thiolen, Epoxy und primären und sekundären Aminen ausgewählt ist.

17. Zusammensetzung nach einem der Ansprüche 13 bis 15, **dadurch gekennzeichnet, dass** das Polysiloxanmakromer ein Polysiloxan ist, das der folgenden allgemeinen Formel (III) entspricht:
T-(CH₂)ₛ-Si-[-(OSiR⁵R⁶)ₜ-R⁷]_{y} (III),
worin T unter NH₂, NHR', Epoxy, OH und SH ausgewählt ist; R⁵, R⁶, R⁷ und R' unabhängig voneinander C₁₋₆-Alkyl, Phenyl, Benzyl, C₆₋₁₂-Alkylphenyl oder Wasserstoff bedeuten; s eine Zahl von 2 bis 100; t eine Zahl von 0 bis 1 000; und y eine Zahl von 1 bis 3 ist.

18. Zusammensetzung nach Anspruch 1, **dadurch gekennzeichnet, dass** sie mindestens ein gepfropftes Siliconpolymer mit einem Polysiloxangerüst enthält, das mit nicht siliconhaltigen, organischen Monomeren gepfropft ist, wobei eine Polysiloxanhauptkette enthalten ist, auf die in der Kette sowie gegebenenfalls an mindestens einem ihrer Enden mindestens eine organische Gruppe gepfropft ist, die kein Silicon enthält.

19. Zusammensetzung nach Anspruch 18, **dadurch gekennzeichnet, dass** das Polymer mit einem Polysiloxangerüst, das mit nicht siliconhaltigen organischen Monomeren gepfropft ist, durch radikalische Copolymerisation mindestens eines nicht siliconhaltigen, anionischen, organischen Monomers, das eine ethylenische Doppelbindung aufweist, und/oder eines nicht siliconhaltigen, hydrophoben, organischen Monomers, das eine ethylenische Doppelbindung aufweist, und eines Polysiloxans, das in seiner Kette mindestens eine funktionelle Gruppe enthält, die mit den ethylenischen Doppelbindungen der nicht siliconhaltigen Monomere reagieren kann, hergestellt werden kann.

20. Zusammensetzung nach Anspruch 19, **dadurch gekennzeichnet, dass** das anionische, organische Monomer mit ethylenischer Doppelbindung einzeln oder in Form von Monomerengemischen unter den geradkettigen oder verzweigten, ungesättigten Carbonsäuren ausgewählt ist.

21. Zusammensetzung nach Anspruch 20**, dadurch gekennzeichnet, dass** das anionische, organische Monomer mit ethylenischer Doppelbindung einzeln oder in Form von Monomerengemischen unter Acrylsäure, Methacrylsäure, Maleinsäure, Maleinsäureanhydrid, Itaconsäure, Fumarsäure und Crotonsäure oder deren Alkalisalzen, Erdalkalisalzen oder Ammoniumsalzen oder deren Gemischen ausgewählt ist.

22. Zusammensetzung nach Anspruch 21, **dadurch gekennzeichnet, dass** das hydrophobe, organische Monomer mit ethylenischer Doppelbindung einzeln oder in Form von Monomerengemischen unter den Estern von Acrylsäure und Alkanolen und/oder Estern von Methacrylsäure und Alkanolen oder deren Gemischen ausgewählt ist, wobei die Alkanole vorzugsweise 1 bis 18 Kohlenstoffatome aufweisen.

23. Zusammensetzung nach Anspruch 22, **dadurch gekennzeichnet, dass** das hydrophobe, organische Monomer mit ethylenischer Doppelbindung einzeln oder in Form von Monomerengemischen unter Isooctyl(meth)acrylat, Isononyl(meth)acrylat, 2-Ethylhexyl(meth)-acrylat, Lauryl(meth)acrylat, Isopentyl(meth)acrylat, n-Butyl(meth)-acrylat, Isobutyl(meth)acrylat, Methyl(meth)acrylat, t-Butyl-(meth)acrylat, Tridecyl(meth)acrylat und Stearyl(meth)acrylat ausgewählt ist.

24. Zusammensetzung nach einem der Ansprüche 18 bis 23, **dadurch gekennzeichnet, dass** das gepfropfte Siliconpolymer an der Siliconhauptkette mindestens eine organische Gruppe mit anionischen Eigenschaften enthält, die durch radikalische (Homo)polymerisation mindestens eines anionischen Monomers vom Typ einer ungesättigten, ganz oder teilweise in Form eines Salzes neutralisierten Carbonsäure erhalten wird.

25. Zusammensetzung nach einem der Ansprüche 18 bis 24, **dadurch gekennzeichnet, dass** das gepfropfte Siliconpolymer unter den Siliconpolymeren ausgewählt ist, die in ihrer Struktur die Einheit der folgenden Formel (IV) aufweisen: 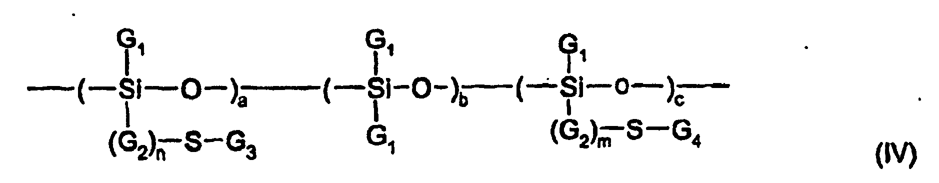 worin die Gruppen G₁, die identisch oder voneinander verschieden sind, Wasserstoff, C₁₋₁₀-Alkyl oder Phenyl; die Gruppen G₂, die identisch oder voneinander verschieden sind, C₁₋₁₀-Alkylen; G₃ eine Polymergruppe, die aus der (Homo)polymerisation mindestens eines anionischen Monomers mit ethylenischer Doppelbindung resultiert; G₄ eine Polymergruppe, die aus der (Homo)polymerisation mindestens eines hydrophoben Monomers mit ethylenischer Doppelbindung resultiert; m und n 0 oder 1; a 0 oder eine ganze Zahl im Bereich von 1 bis 50; b eine ganze Zahl im Bereich von 10 bis 350; und c 0 oder eine ganze Zahl von 1 bis 50 bedeuten, mit der Maßgabe, dass einer der Parameter a oder c von 0 verschieden ist.

26. Zusammensetzung nach Anspruch 25, **dadurch gekennzeichnet, dass** die Einheit der Formel (IV) mindestens eine der folgenden Eigenschaften aufweist:
- die Gruppen G₁ bedeuten C₁₋₁₀-Alkyl;
- n ist nicht Null und die Gruppen G₂ bedeuten eine zweiwertige Gruppe mit 1 bis 3 Kohlenstoffatomen;
- G₃ bedeutet eine Polymergruppe, die aus der (Homo)polymerisation mindestens eines Monomers vom Typ einer Carbonsäure mit ethylenischer Doppelbindung resultiert;
- G₄ bedeutet eine Polymergruppe, die aus der (Homo)polymerisation mindestens eines Monomers vom Typ C₁₋₁₀-Alkyl(meth)-acrylat resultiert.

27. Zusammensetzung nach Anspruch 25 oder 26, **dadurch gekennzeichnet, dass** die Einheit der Formel (IV) gleichzeitig die folgenden Eigenschaften aufweist:
- die Gruppen G₁ bedeuten Methyl;
- n ist nicht Null und die Gruppen G₂ bedeuten Propylen;
- G₃ bedeutet eine Polymergruppe, die aus der (Homo)polymerisation von zumindest Acrylsäure und/oder Methacrylsäure resultiert;
- G₄ bedeutet eine Polymergruppe, die aus der (Homo)polymerisation mindestens eines Monomers vom Typ Isobutyl(meth)-acrylat oder Methyl(meth)acrylat resultiert.

28. Zusammensetzung nach einem der Ansprüche 18 bis 27, **dadurch gekennzeichnet, dass** das Zahlenmittel des Molekulargewichts des gepfropften Polymers mit Polysiloxangerüst, das mit nicht siliconhaltigen, organischen Monomeren gepfropft ist, im Bereich von etwa10 000 bis 1 000 000 und noch bevorzugter etwa 10 000 bis 100 000 liegt.

29. Zusammensetzung nach einem der vorhergehenden Ansprüche, **dadurch gekennzeichnet, dass** das kationische Polymer unter den quartären Celluloseetherderivaten, Cyclopolymeren, kationischen Polysacchariden und deren Gemischen ausgewählt ist.

30. Zusammensetzung nach Anspruch 29, **dadurch gekennzeichnet, dass** das Cyclopolymer unter den Copolymeren von Dimethyldiallyl-ammoniumchlorid und Acrylamid ausgewählt ist.

31. Zusammensetzung nach Anspruch 29, **dadurch gekennzeichnet, dass** die quartären Celluloseetherderivate unter den Hydroxyethylcellulosen ausgewählt ist, die mit einem mit einer Trimethylammoniumgruppe substituierten Epoxid umgesetzt wurden.

32. Zusammensetzung nach Anspruch 29, **dadurch gekennzeichnet, dass** die kationischen Polysaccharide unter den mit einem 2,3-Epoxypropyltrimethylammoniumsalz modifizierten Guargummen ausgewählt sind.

33. Zusammensetzung nach einem der vorhergehenden Ansprüche, **dadurch gekennzeichnet, dass** das aminierte Silicon ausgewählt ist unter:
a) Polysiloxanen, die gemäß CTFA-Nomenklatur als "Amodimethicone" bezeichnet werden und der folgenden Formel entsprechen: 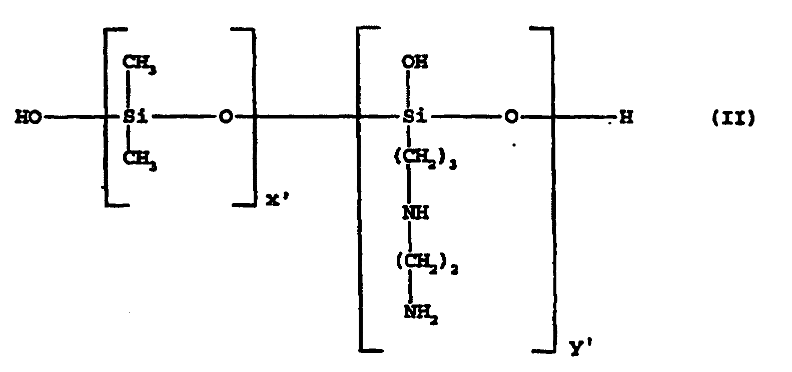 worin x' und y' ganze Zahlen bedeuten, die vom Molekulargewicht abhängen und im allgemeinen so gewählt sind, dass das Zahlenmittel des Molekulargewichts im Bereich von etwa 5 000 bis 500 000 liegt;
(b) kationische Siliconpolymere der folgenden Formel:
R'ₐG₃₋ₐ-Si(OSiG₂)ₙ-(OSiG_{b}R'_{2-b})ₘ-O-SiG₃₋ₐ-R'ₐ (III)
worin bedeuten:
G Wasserstoff, Phenyl, OH, C₁₋₈-Alkyl, beispielsweise Methyl,
a 0 oder eine ganze Zahl von 1 bis 3 und insbesondere 0,
b 0 oder 1 und insbesondere 1,
m und n Zahlen, die so ausgewählt sind, dass die Summe (n + m) insbesondere im Bereich von 1 bis 2000 und besonders 50 bis 150 liegen kann, wobei n eine Zahl von 0 bis 1999 und insbesondere 49 bis 149 und m eine Zahl von 1 bis 2000 und insbesondere 1 bis 10 bedeuten kann,
die Gruppe R' eine einwertige Gruppe der Formel -C_{q}H_{2q}L, worin q eine Zahl von 1 bis 8 ist und L eine gegebenenfalls quaternisierte aminierte Gruppe bedeutet, welche unter den folgenden Gruppen ausgewählt ist:
- NR"-CH₂-CH₂-N'(R")₂
- N(R")₂
- N^{⊕}(R")₃A-
- N(R")-CH₂-CH₂-N^{⊕}R"H₂A-,
worin R" Wasserstoff, Phenyl, Benzyl oder eine einwertige gesättigte Kohlenwasserstoffgruppe, beispielsweise eine Alkylgruppe mit 1 bis 20 Kohlenstoffatomen bedeuten kann und A- ein Halogenid bedeutet, wie beispielsweise Fluorid, Chlorid, Bromid oder Iodid.
(c) kationische Siliconpolymere der folgenden Formel: 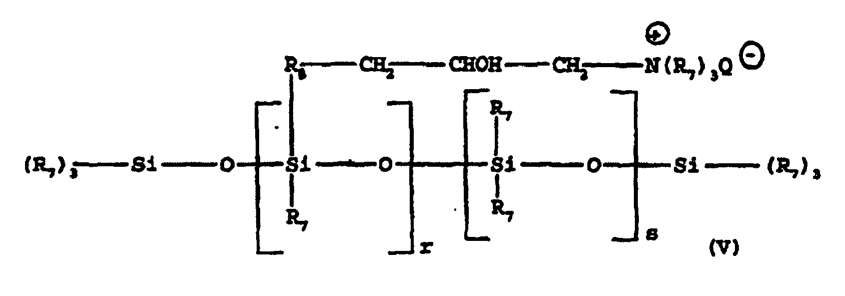 worin bedeuten:
R₇ eine einwertige Kohlenwasserstoffgruppe mit 1 bis 18 Kohlenstoffatomen und insbesondere C₁₋₁₈-Alkyl oder C₂₋₁₈-Alkenyl, beispielsweise Methyl;
R₈ eine zweiwertige Kohlenwasserstoffgruppe, insbesondere eine C₁₋₁₈-Alkylengruppe oder eine zweiwertige C₁₋₁₈-Alkylenoxygruppe, beispielsweise eine Gruppe mit 1 bis 8 Kohlenstoffatomen;
Q⁻ ein Halogenid, insbesondere Chlorid;
r einen statistischen Mittelwert von 2 bis 20 und insbesondere 2 bis 8; und
s einen statistischen Mittelwert von 20 bis 200 und insbesondere 20 bis 50.

34. Zusammensetzung nach einem der vorhergehenden Ansprüche, **dadurch gekennzeichnet, dass** die reinigende Basisformulierung in einem Gewichtsanteil von 4 bis 50 %, bezogen auf das Gesamtgewicht der Zusammensetzung, vorliegt.

35. Zusammensetzung nach Anspruch 34, **dadurch gekennzeichnet, dass** der Gewichtsanteil im Bereich von 10 bis 35 % liegt.

36. Zusammensetzung nach Anspruch 35, **dadurch gekennzeichnet, dass** der Gewichtsanteil im Bereich von 12 bis 25 % liegt.

37. Zusammensetzung nach einem der vorhergehenden Ansprüche, **dadurch gekennzeichnet, dass** das kationische Polymer in einem Gewichtsanteil von 0,001 bis 10 %, bezogen auf das Gesamtgewicht der Zusammensetzung, vorliegt.

38. Zusammensetzung nach Anspruch 37, **dadurch gekennzeichnet, dass** der Mengenanteil im Bereich von 0,005 bis 5 % liegt.

39. Zusammensetzung nach Anspruch 38, **dadurch gekennzeichnet, dass** der Mengenanteil im Bereich von 0,01 bis 3 % liegt.

40. Zusammensetzung nach einem der vorhergehenden Ansprüche, **dadurch gekennzeichnet, dass** das gepfropfte Siliconpolymer in einem Gewichtsanteil von 0,01 bis 20 %, bezogen auf das Gesamtgewicht der Zusammensetzung, vorliegt.

41. Zusammensetzung nach Anspruch 40, **dadurch gekennzeichnet, dass** der Mengenanteil im Bereich von 0,1 bis 15 % liegt.

42. Zusammensetzung nach Anspruch 41, **dadurch gekennzeichnet, dass** der Mengenanteil im Bereich von 0,5 bis 10 % liegt.

43. Zusammensetzung nach einem der vorhergehenden Ansprüche, **dadurch gekennzeichnet, dass** das aminierte Silicon in einem Gewichtsanteil von 0,05 bis 10 %, bezogen auf das Gesamtgewicht der Zusammensetzung, vorliegt.

44. Zusammensetzung nach Anspruch 43, **dadurch gekennzeichnet, dass** der Mengenanteil im Bereich von 0,1 bis 5 %, bezogen auf das Gesamtgewicht der Zusammensetzung, liegt.

45. Zusammensetzung nach einem der vorhergehenden Ansprüche, **dadurch gekennzeichnet, dass** sie eine pH-Wert im Bereich von 3 bis 10 aufweisen.

46. Verwendung einer Zusammensetzung nach einem der vorhergehenden Ansprüche zur Reinigung und/oder zum Frisieren und/oder zum Konditionieren der Haare.

47. Verwendung eines aminierten Silicons, um die Frisierwirkung einer reinigenden Zusammensetzung für das Haar zu verbessern, die mindestens ein kationisches Polymer und mindestens ein gepfropftes Siliconpolymer nach einem der Ansprüche 1 bis 28 enthält.

48. Verfahren zum Waschen und Konditionieren von Keratinfasern, wie den Haaren, das darin besteht, auf die feuchten Fasern eine wirksame Menge einer Zusammensetzung nach einem der Ansprüche 1 bis 45 aufzutragen und anschließend, gegebenenfalls nach einer Einwirkzeit, mit Wasser zu spülen.
